(19) Europäisches Patentamt
European Patent Office
Office européen des brevets

(11) EP 1 954 678 B1

(12) **EUROPEAN PATENT SPECIFICATION**

(45) Date of publication and mention of the grant of the patent:
**21.03.2012 Bulletin 2012/12**

(21) Application number: **06830200.9**

(22) Date of filing: **30.11.2006**

(51) Int Cl.:
***C07D 213/38*** *(2006.01)* ***C07D 295/12*** *(2006.01)*
***C07D 307/52*** *(2006.01)* ***C07D 307/81*** *(2006.01)*
***C07D 317/58*** *(2006.01)* ***C07D 333/20*** *(2006.01)*
***C07D 333/28*** *(2006.01)* ***C07D 333/58*** *(2006.01)*
***C07D 407/06*** *(2006.01)* ***C07D 409/06*** *(2006.01)*
***A61K 31/4965*** *(2006.01)* ***A61K 31/497*** *(2006.01)*
***A61P 1/00*** *(2006.01)*

(86) International application number:
**PCT/EP2006/069087**

(87) International publication number:
**WO 2007/063086 (07.06.2007 Gazette 2007/23)**

(54) **NOVEL NK1 AND NK2 ANTAGONISTS**

NEUE NK1- UND NK2-ANTAGONISTEN

NOUVEAUX ANTAGONISTES DE NK1 ET DE NK2

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.11.2005 EP 05111490**

(43) Date of publication of application:
**13.08.2008 Bulletin 2008/33**

(73) Proprietor: **Abbott Products GmbH**
**30173 Hannover (DE)**

(72) Inventors:
- **JASSERAND, Daniel**
**30173 Hannover (DE)**
- **SCHOEN, Uwe**
**30173 Hannover (DE)**
- **ANTEL, Jochen**
**30173 Hannover (DE)**
- **FIRNGES, Michael**
**30173 Hannover (DE)**
- **REICHE, Dania**
**30173 Hannover (DE)**
- **SANN, Holger**
**30173 Hannover (DE)**
- **BRÜCKNER, Reinhard**
**30173 Hannover (DE)**

(74) Representative: **Bensiek, Stephan et al**
**ABPH-ZP**
**Abbott Products GmbH**
**Hans-Böckler-Allee 20**
**30173 Hannover (DE)**

(56) References cited:
**EP-A- 0 428 434** **EP-A- 1 293 506**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 954 678 B1

## Description

Introduction

The present invention relates to novel 3-cyano-naphthalene-1-carboxylic acid perhydroxyalkylmethyl-piperazine compounds of formula I which are antagonistic to tachykinin receptors. The present invention is further directed to pharmaceuticals compositions comprising such compounds, to processes for the preparation of such compounds and to intermediate products of these processes.

Background

The tachykinins include the naturally-occurring neuropeptides substance P, neurokinin A and neurokinin B. The tachykinins act as agonists of receptors occurring in larger mammals and humans, such as the neurokinin (= NK) 1 receptor, the NK2 receptor and the NK3 receptor. Artificially prepared compounds which are antagonistic to tachykinin receptors are usually classified according to their relative ability to bind to one or more of the aforementioned three receptor subtypes. In the physiological process the tachykinins play e.g. an important part in the transmission of pain, emesis, neurogenic inflammations, bladder inflammation, inflammatory joint diseases or asthmatic complaints.

Inter alia, piperazine derivatives which act as antagonists to the $NK_1$ receptor are already known from WO 2004/033 428 A1.

Inter alia, piperazine derivatives which act as antagonists to the $NK_2$ receptor are already known from EP 1 293 506 A1.

Further piperazine derivatives which can act as antagonists to tachykinin receptors are known from WO 96/10 568.

It was an object of the present invention to provide novel active substances having properties antagonistic to tachykinin receptors $NK_1$ and $NK_2$ and an improved action profile, which are suitable in particular for the treatment and/or prophylaxis of respiratory diseases, in particular asthma, bronchitis, cough, and rhinitis; skin diseases, in particular inflammatory skin reactions, allergic skin reactions, and psoriasis; arthropathy diseases, in particular arthtitis, vasculitides and systemic lupus erythematosus; functional or inflammatory disorders in the gastrointestinal tract, in particular pseudomembranous colitis and diarrhoe; bleb diseases such as cystitis and interstitial cystitis; and migraine. The compounds of the present invention are particular suitable for the treatment of peripheral disorders such as functional and inflammatory disorders of the gastrointestinal tract, such as IBS.

It has now been found, surprisingly, that a group of novel 3-cyano-naphthalene-1-carboxylic acid perhydroxyalkylmethyl-piperazine compounds is distinguished by properties antagonistic to tachykinin receptors, in particular to and $NK_1$- and $NK_2$ receptors, and has a marked action component directed at the peripheral region. Accordingly, the group of compounds according to the invention appears particularly suitable for the treatment of peripheral disorders in which tachykinins, in particular neurokinin A, participate as transfer agents, for example for the treatment and/or prophylaxis of respiratory diseases, in particular asthma, bronchitis, cough, and rhinitis; skin diseases, in particular inflammatory skin reactions, allergic skin reactions, and psoriasis; arthropathy diseases, in particular arthritis, vasculitides and systemic lupus erythematosus; functional or inflammatory disorders in the gastrointestinal tract, in particular pseudomembranous colitis and diarrhoe; bleb diseases such as cystitis and interstitial cystitis; and migraine.

Another advantage of the compounds of the present invention is their very balanced combined $NK_1$- and $NK_2$-profile.

Another advantage of the compounds of the present invention is the synergistic effect between the $NK_1$- and $NK_2$-profile.

Summary

The subject of the invention are novel 3-cyano-naphthalene-1-carboxylic acid perhydroxyalkylmethyl-piperazine compounds of the general formula I,

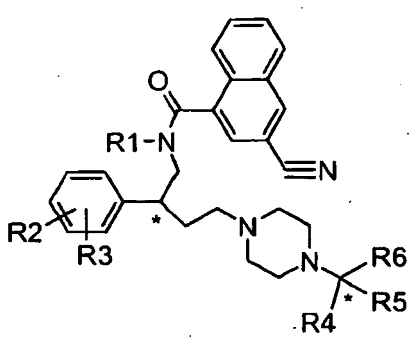

wherein

R1 is selected from the group consisting of: hydrogen and $C_1$ to $C_4$ alkyl,

R2 is halogen,

R3 is halogen,

R4 is selected from the group consisting of: 2-furanyl, 3-furanyl, 2-thiophene, 3-thiophene, phenyl, benzyl, 2-benzofuranyl, 3-benzofuranyl, 5-chloro-2-thiophene, 4-methylphenyl, 3,4-methylenedioxyphenyl, 2-methoxyphenyl, 4-methoxyphenyl, 2-pyridinyl, 3-pyridinyl, 1-benzo[c]thiophene, 4-benzo[c]thiophene, 5-benzo[c]thiophene, 2-benzo[b]thiophene, 3-benzo[b]thiophene, 4-benzo[b]thiophene, 5-benzo[b]thiophene, 6-benzo[b]thiophene, 7-benzo[b]thiophene, 1-benzo[1,3]dioxole, 4-benzo[1,3]dioxole, and 5-benzo[1,3]dioxole,

R5 is selected from the group consisting of: hydrogen and R6,

R6 represents a subgroup of the general formula

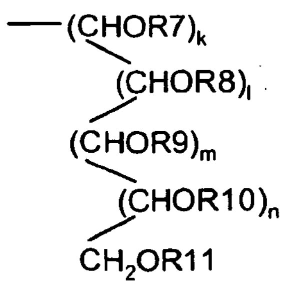

wherein

R7 is selected from the group consisting of: hydrogen or $C_1$ to $C_4$ alkanoyl, or together with another substituent, selected from the group consisting of R8, R9, R10 and R11, may form a 5- or 6-ring bridged by carbonyl, or by methylene optionally substituted by $C_1$ to $C_4$ alkyl or $C_4$ to $C_5$ alkylene,

R8 is selected from the group consisting of: hydrogen or $C_1$ to $C_4$ alkanoyl, or together with another substituent, selected from the group consisting of R7, R9, R10 and R11, may form a 5- or 6-ring bridged by carbonyl, or by methylene optionally substituted by $C_1$ to $C_4$ alkyl or $C_4$ to $C_5$alkylene,

R9 is selected from the group consisting of: hydrogen or $C_1$ to $C_4$ alkanoyl, or together with another substituent, selected from the group consisting of R7, R8, R10 and R11, may form a 5- or 6-ring bridged by carbonyl, or by methylene optionally substituted by $C_1$ to $C_4$ alkyl or $C_4$ to $C_5$ alkylene,

R10 is selected from the group consisting of: hydrogen or $C_1$ to $C_4$ alkanoyl, or together with another substituent,

selected from the group consisting of R7, R8, R9 and R11, may form a 5- or 6-ring bridged by carbonyl, or by methylene optionally substituted by $C_1$ to $C_4$ alkyl or $C_4$ to $C_5$ alkylene,

R11 is selected from the group consisting of: hydrogen or $C_1$ to $C_4$ alkanoyl, or together with another substituent, selected from the group consisting of R7, R8, R9 and R10, may form a 5- or 6-ring bridged by carbonyl, or by methylene optionally substituted by $C_1$ to $C_4$ alkyl or $C_4$ to $C_5$ alkylene,

k is 0 or 1,

l is 0 or 1,

m is 0 or 1,

n is 0 or 1,

or physiologically compatible acid addition salts thereof.

Furthermore, subjects of the invention are pharmaceutical compositions comprising the compounds of formula I. Furthermore, subjects of the invention are processes for the preparation of the compounds of formula I and intermediate products of these processes.

Detailed description

The term pharmaceutical compositions as used in the present invention means pharmaceutical compositions comprising a pharmacologically effective quantity of a compound of the present invention and pharmaceutical auxiliaries and/or carriers conventional in pharmaceutical compositions.

The subject of the invention are novel 3-cyano-naphthalene-1-carboxylic acid perhydroxyalkylmethyl-piperazine compounds of the general formula I as described above. Where substituents in compounds of formula I stand for halogen, fluorine, chlorine or bromine are suitable. Chlorine is preferred. The designation (hetero)aryl is to be understood within the scope of the present invention as possibly comprising both aryl and heteroaryl radicals.

In a preferred embodiment of the present invention, R1 represents methyl.

In another preferred embodiment of the present invention, R2 and R3 each represent chlorine.

In another preferred embodiment of the present invention, R4 is selected from the group consisting of: 2-furanyl, 3-furanyl, 2-thiophene, 3-thiophene, phenyl, benzyl, 2-benzofuranyl, 5-chloro-2-thiophene, 4-methylphenyl, 3,4-methylenedioxyphenyl, 2-methoxyphenyl and 4-methoxyphenyl.

In another preferred embodiment of the present invention, R4 is selected from the group consisting of: 2-furanyl, 3-furanyl, 2-thiophene, and 3-thiophene.

In another preferred embodiment of the present invention, R5 represents hydrogen.

In another preferred embodiment of the present invention, R7 and R11 each represent hydrogen; k represents 1; and l, m, n are each 0.

In another preferred embodiment of the present invention, R7, R8 and R11 each represent hydrogen; k and l are each 1; and m and n are each 0.

In another preferred embodiment of the present invention, R7, R8, R9 and R11 each represent hydrogen; k, l and m are each 1; and n is 0.

In another preferred embodiment of the present invention, R7 to R11 each represent hydrogen; k, l and m are each 1; and n is 0.

In another preferred embodiment of the present invention, the chiral centre *C is in the S configuration.

Where a substituent covered by the subgroup $R^6$ from the group consisting of $R^7$, $R^8$, $R^9$, $R^{10}$ and $R^{11}$ together with another substituent selected from this group stands for a 5- or 6-ring bridged by methylene optionally substituted by $C_1$ to $C_4$ alkyl or $C_4$ to $C_5$ alkylene, in particular 5- or 6-rings bridged by methylene, 1,1-dimethylmethylene, 1,1-*spiro*-tetramethylene-methylene or 1,1-*spiro*-pentamethylene-methylene are suitable. Corresponding 5- or 6-rings bridged by carbonyl are to be regarded as cyclic carbonates. k preferably stands for 1. n preferably stands for 0. $R^6$ thus preferably represents an optionally substituted 1,2-diol radical, a 1,2,3-triol radical or a 1,2,3,4-tetrol radical. The carbon atoms bearing the substituents $R^8$, $R^9$, $R^{10}$ and $R^{11}$ are asymmetric and may each occur in two different configurations. Due to this, $R^7$ may occur in several stereoisomeric forms. The present invention also covers, in addition to the compounds of formula I which contain mixtures of stereoisomeric forms of the subgroup $R^7$, compounds of formula I in which isomerically pure subgroups $R^7$ are contained. Preferred subgroups $R^7$ are *xylo*-1,2,3,4-tetrahydroxybutyl, *lyxo*-1,2,3,4-tetrahydroxybutyl, *arabino*-1,2,3,4-tetrahydroxybutyl, *threo*-1,2,3-trihydroxypropyl, *erythro*-1,2,3-trihydroxypropyl and *glycero*-1,2-dihydroxyethyl. The carbohydrates selected from the D-series of the carbohydrates on which the subgroups

$R^7$ are based mostly produce the most beneficial results. Diastereomerically pure subgroups $R^7$ are preferred.

Particularly preferred compounds of formula I are selected from the group consisting of:

3-cyano-*N*-[(2*S*)-2-(3,4-dichlorophenyl)-4-{4-[(1*R*,2*S*)-1-(2-furyl)-2,3-dihydroxypropyl]piperazin-1-yl}butyl]-*N*-methyl-1-naphthamide;

(2*S*,3*R*)-2-(acetyloxy)-3-{4-[(3*S*)-4-[3-cyano-1-naphthoyl)(methyl)amino]-3-(3,4-dichlorophenyl)butyl]piperazin-1-yl}-3-(2-furyl)propyl acetate;

3-cyano-*N*-[(2*S*)-2-(3,4-dichlorophenyl)-4-{4-[1-(2-furyl)-2-hydroxyethyl]piperazin-1-yl}butyl]-*N*-methyl-1-naphthamide;

3-cyano-*N*-[(2*S*)-2-(3,4-dichlorophenyl)-4-{4-[(1*R*,2*S*,3*R*,4*R*)-1-(2-furyl)-2,3,4,5-tetrahydroxypentyl]piperazin-1-yl}butyl]-*N*-methyl-1-naphthamide;

3-cyano-*N*-[(2*S*)-2-(3,4-dichlorophenyl)-4-{4-[(1*R*,2*S*,3*S*,4*R*)-1-(2-furyl)-2,3,4,5-tetrahydroxypentyl]piperazin-1-yl}butyl]-*N*-methyl-1-naphthamide;

3-cyano-*N*-[(2*S*)-2-(3,4-dichlorophenyl)-4-{4-[(1*S*,2*S*,3*S*,4*R*)-2,3,4,5-tetrahydroxy-1-(3-thienyl)pentyl]piperazin-1-yl}butyl]-*N*-methyl-1-naphthamide;

3-cyano-*N*-[(2*S*)-2-(3,4-dichlorophenyl)-4-{4-[(1*S*,2*S*,3*R*,4*R*)-2,3,4,5-tetrahydroxy-1-(3-thienyl)pentyl]piperazin-1-yl}butyl]-*N*-methyl-1-naphthamide;

3-cyano-*N*-[(2*S*)-2-(3,4-dichlorophenyl)-4-{4-[1-(2-furyl)-2-hydroxy-1-(hydroxymethyl)ethyl]piperazin-1-yl}butyl]-*N*-methyl-1-naphthamide;

3-cyano-*N*-[(2*S*)-2-(3,4-dichlorophenyl)-4-{4-[1-(2-furyl)-2-hydroxy-1-(hydroxymethyl)ethyl]piperazin-1-yl}butyl]-*N*-methyl-1-naphthamide;

3-cyano-*N*-[(2*S*)-2-(3,4-dichlorophenyl)-4-{4-[(1*S*,2*S*)-2,3-dihydroxy-1-(3-thienyl)propyl]piperazin-1-yl}butyl]-*N*-methyl-1-naphthamide;

3-cyano-N-[(2*S*)-2-(3,4-dichlorophenyl)-4-{4-[(1*R*,2*S*)-2,3-dihydroxy-1-(2-thienyl)propyl]piperazin-1-yl}butyl]-*N*-methyl-1-naphthamide;

3-cyano-*N*-[(2*S*)-2-(3,4-dichlorophenyl)-4-{4-[(*S*)-[(4*S*)-2-oxo-1,3-dioxolan-4-yl](3-thienyl)methyl]piperazin-1-yl}butyl]-*N*-methyl-1-naphthamide;

3-cya no-*N*-[(2*S*)-2-(3,4-dichlorophenyl)-4-(4-{(*R*)-2-furyl[(4*S*)-2-oxo-1,3-dioxolan-4-yl]methyl}piperazin-1-yl)butyl]-*N*-methyl-1-naphthamide;

3-cyano-*N*-[(2*S*)-2-(3,4-dichlorophenyl)-4-{4-[(1*S*,2*S*)-1-(3-furyl)-2,3-dihydroxypropyl]piperazin-1-yl}butyl]-*N*-methyl-1-naphthamide;

3-cyano-*N*-[(2*S*)-2-(3,4-dichlorophenyl)-4-{4-[1-(2-furyl)-2,3-dihydroxypropyl]piperazin-1-yl}butyl]-*N*-methyl-1-naphthamide;

3-cyano-*N*-[(2*S*)-2-(3,4-dichlorophenyl)-4-{4-[(1*S*,2*S*)-2,3-dihydroxy-1-phenylpropyl]piperazin-1-yl)butyl]-*N*-methyl-1-naphthamide;

3-cyano-*N*-[(2)-2-(3,4-dichlorophenyl)-4-{4-[(1*S*,2*S*)-2,3-dihydroxy-1-(4-methoxyohenyl)propyl]piperazin-1-yl}butyl]-*N*-methyl-1-naphthamide;

*N*-[(2*S*)-4-{4-[(1*R*,2*S*)-1-(1-benzofuran-2-yl)-2,3-dihydroxypropyl]piperazin-1-yl}-2-(3,4-dichlorophenyl)butyl]-3-cyano-*N*-methyl-1-naphthamide;

*N*-[(2*S*)-4-{4-[(1*R*,2*S*)-1-(5-chloro-2-thienyl)-2,3-dihydroxypropyl]piperazin-1-yl}-2-(3,4-dichlorophenyl)butyl]-3-cyano-*N*-methyl-1-naphthamide;

*N*-[(2*S*)-4-{4-[(1*S*,2*S*)-1-(1,3-benzodioxol-5-yl)-2,3-dihydroxypropyl]piperazin-1-yl}-2-(3,4-dichlorophenyl)butyl]-3-cyano-*N*-methyl-1-naphthamide; and

N-[(2*S*)-4-{4-[(1*R*,2*S*)-1-(1-benzothien-2-yl)-2,3-dihydroxypropyl]piperazin-1-yl}-2-(3,4-dichlorophenyl)butyl]-3-cyano-*N*-methyl-1-naphthamide.

Process 1: The compounds of the present invention may be prepared by reacting a compound of formula II

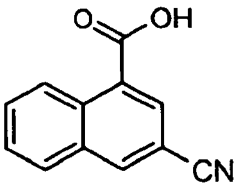

II

with a chlorine source, preferably oxalyl dichloride, to give a compound of formula III,

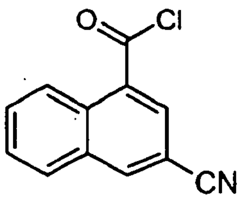

III

The compound of formula III is reacted with a compound of the general formula IV,

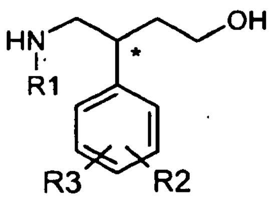

IV

to give a compound of the general formula V

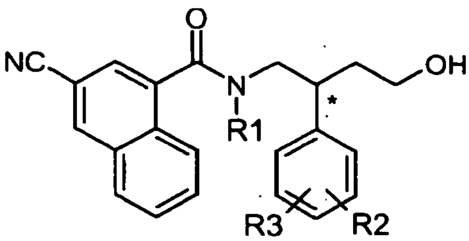

V

wherein $R^1$, $R^2$ and $R^3$ have the meaning as defined above. The compound of general formula V is reacted with methanesulfonyl chloride to give a compound of the general formula VI,

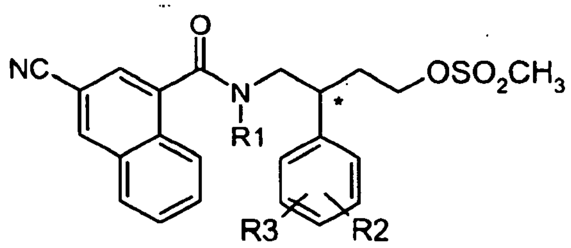

VI

wherein $R^1$, $R^2$ and $R^3$ have the meaning as defined above. The compound of general formula VI is first reacted with an alkali metal halide MX wherein M stands for an alkali metal, preferably sodium and wherein X stands for halogen, preferably iodide, and subsequently reacted with a compound of general formula XIX

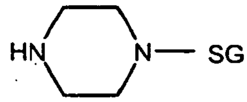

XIX

wherein SG stands for a cleavable protective group, preferably tertiary butoxycarbonyl, to give a compound of the general formula VIa,

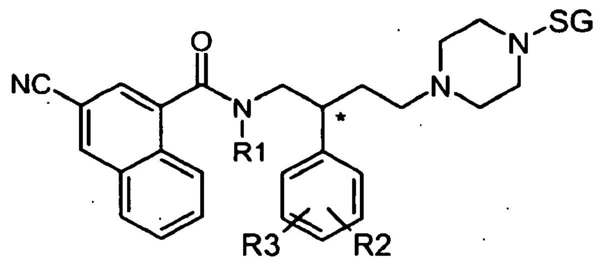

VIa

wherein $R^1$, $R^2$ and $R^3$ have the meaning as defined above. The compound of general formula VIa is hydrolyzed in an acidic medium to give a compound of the general formula VII

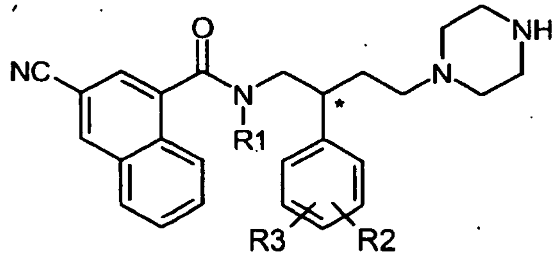

VII

wherein $R^1$, $R^2$ and $R^3$ have the meaning as defined above. The compound of general formula VII is reacted with a compound of the general formula VIII

$R4 - B(OH)_2$ VIII

and a compound of the general formula IX,

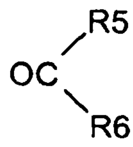

IX

to result in a compound of general formula I which is optionally converted into its physiologically compatible acid addition salt, wherein $R^1$, $R^2$, $R^3$, $R^4$, $R^5$ and $R^6$ have the meaning as defined above.

Process 2: The compounds of the present invention may be also prepared by reacting a compound of the general formula X

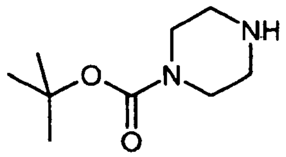

X

with a compound of the general formula VIII

$R4 - B(OH)_2$ VIII

and a compound of the general formula IX,

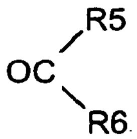

IX

to give a compound of general formula XI

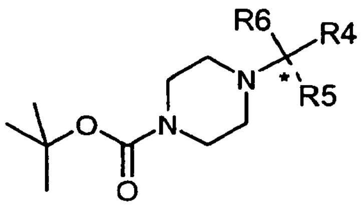

XI

wherein $R^4$, $R^5$ and $R^6$ have the meaning as defined above. The compound of general formula XI is then hydrolyzed in an acidic medium to give a compound of the general formula XII,

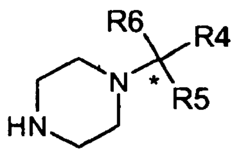

XII

wherein $R^4$, $R^5$ and $R^6$ have the meaning as defined above. The compound of general formula XII is reacted with a compound of general formula XIII,

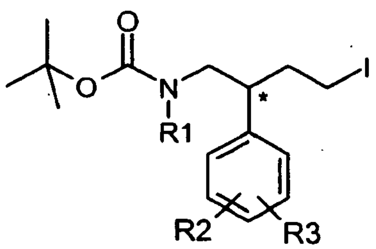

XIII

to give a compound of general formula XIV,

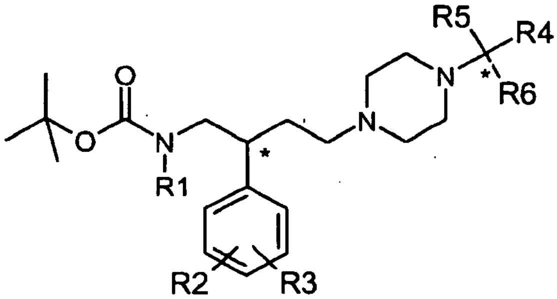

XIV

wherein $R^1$, $R^2$, $R^3$, $R^4$, $R^5$ and $R^6$ have the meaning as defined above. The compound of general formula XIV is reacted then hydrolyzed in an acidic medium to give a compound of general formula XV,

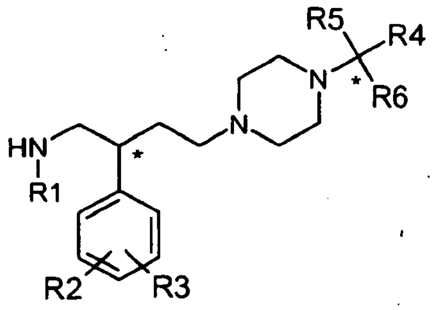

XV

wherein $R^1$, $R^2$, $R^3$, $R^4$, $R^5$ and $R^6$ have the meaning as defined above. The compound of general formula XV is then reacted with a compound of formula III to result in a compound of general formula I which is optionally converted into its physiologically compatible acid addition salt wherein $R^1$, $R^2$, $R^3$, $R^4$, $R^5$ and $R^6$ have the meaning as defined above.

Process 2 may be modified in such a way that the compound of general formula XII,

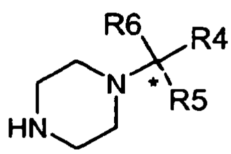

XII

is reacted with a compound of general formula XVI

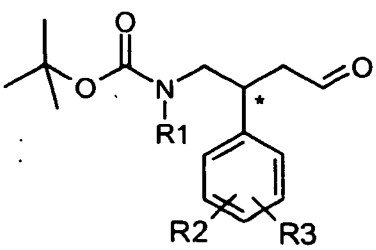

XVI

to give a compound of the general formula XIV wherein $R^1$, $R^2$, $R^3$, $R^4$, $R^5$ and $R^6$ have the meaning as defined above. The compound of the general formula XVI

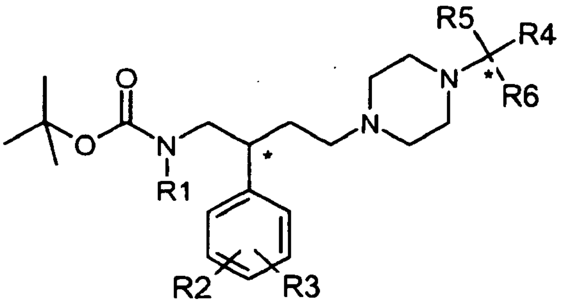

XIV

is then further reacted as previously described.

Process 3: The compounds of the present invention may be also prepared by reacting a compound of the general formula II

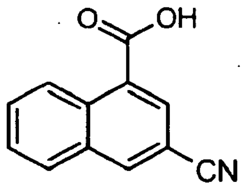

II

with a chlorine source to give a compound of formula III,

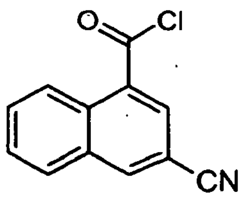

III

The compound of formula III is then reacted with a compound of the general formula IV,

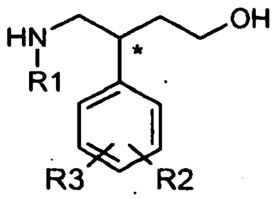

IV

to give a compound of the general formula V,

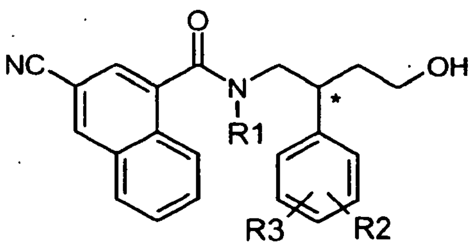

V

The compound of general formula V is oxidized to give a compound of the general formula VI,

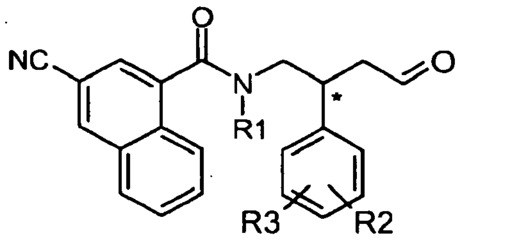

XVII

The compound of general formula XVII is reacted with a compound of general formula XII

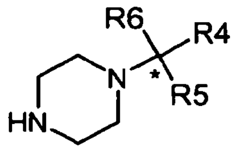

XII

to result in a compound of general formula I which is optionally converted into its physiologically compatible acid addition salt.

The reaction of processes I and III, respectively, in which a compound of general formula VII or X, respectively, is reacted with compounds of general formulae VIII and IX to result in the formation of a compound of general I, can be carried out in known manner under the conditions of a boronic Mannich reaction (cf. e.g. N.A. Petasis et al., Journal of the American Chemical Society 120 (1998) 11798-11799, WO 98/00398 or WO 00/24510). According to this, a compound of formula VII or X can be reacted in the manner of a one-pot reaction with a boronic acid of formula VIII and a carbohydrate of formula IX which is optionally protected by suitable protective groups in a solvent which is inert under the reaction conditions. Suitable protective groups for carbohydrates are known per se, for example, from J.A.W. McOmie, "Protective Groups in Organic Chemistry", Plenum Press, 1973, or from T.W. Green, P.G. Wuts, "Protective Groups in Organic Synthesis", Wiley and Sons, 1999. Suitable solvents are dipolar-protic organic solvents such as lower alkanols, for example straight-chain or branched $C_{1-4}$-alkanols, preferably ethanol, or mixtures of these aforementioned solvents with water or with dipolar-aprotic solvents such as lower haloalkanes, preferably dichloromethane, are suitable. Suitable reaction temperatures are between room temperature and the boiling point of the solvent or of the solvent mixture. The compounds of formulae VII, X, VIII and IX may preferably be combined in succession in this given sequence. Likewise, it is also possible, first to combine a compound of formula VII with a compound of formula IX and then with a compound of formula VII or X. The chiral centre bearing the subgroups R4, R5 and R6 newly produced by this coupling reaction in compounds of formula I is usually formed with a very high degree of diastereo-control as an "anti" product.

The compounds of formula I which bear at least one free hydroxyl group in the subgroup $R^6$ may if desired then also be reacted with carboxylic acids $R^{12}COOH$ of formula XVII, wherein $R^{12}$ has the meaning of straight-chain or branched alkyl with 1 to 3 carbon atoms, whereby the free hydroxyl groups of the subgroup $R^6$ are acylated. Usually under these circumstances peracylation of the free hydroxyl groups of the subgroup $R^6$ takes place. The acids of formula XVII or their reactive derivatives may be used as acylation agents. In particular acid anhydrides and acid halides are suitable reactive derivatives. The acylation may be carried out in an organic solvent which is inert under the reaction conditions, preferably at temperatures between -20°C and room temperature. Suitable solvents are in particular aromatic hydrocarbons such as benzene or toluene, cyclic or open-chain di-lower alkyl ethers such as diethyl ether, tetrahydrofuran (= THF) or dioxane, partially halogenated lower hydrocarbons such as dichloromethane or mixtures of these solvents. Where an acid anhydride or an acid halide of the acids of formula XVII is used as acylation agent, the acylation may expediently take place in the presence of an acid-binding reagent. Suitable acid-binding reagents are non-nucleophilic organic bases soluble in the reaction mixture, such as pyridine, triethylamine or 4-dimethylaminopyridine. Organic bases used in excess can simultaneously also be used as solvents.

The compounds of formula I which bear at least two free hydroxyl groups in the subgroup $R^6$ may if desired, after their preparation described above, also be reacted with a reactive carbonyl-synthesis equivalent, instead of a reaction with compounds of formula XVII, whereby the subgroup $R^6$ can be carbonylated. The reaction can take place in known manner. For example, a compound of formula I can be reacted in an organic solvent which is inert under the reaction conditions. Suitable reactive carbonyl synthesis equivalents are for example phosgene or substances which

react like phosgene, such as bis-(trichloromethyl)carbonate (= triphosgene), trichloromethyl chloroformate (= diphosgene) or in particular carbonyldiimidazole. Expediently an acid-binding reagent may be added to the reaction mixture. Suitable acid-binding reagents are the acid-binding reagents given above for the reaction of compounds of formula I with compounds of formula XVII. Suitable reaction temperatures are between about - 20°C and room temperature.

The compounds of formula I which bear at least two free hydroxyl groups in the subgroup $R^6$ may if desired, after their preparation described above, instead of a reaction with compounds of formula XVII or instead of a reaction with reactive carbonyl synthesis equivalents, also be reacted with a di-lower alkyl ketone or a $C_{5-6}$-cycloalkyl ketone in the subgroup $R^6$, to produce a 5- or 6-ring derivative bridged [by] methylene optionally substituted by lower alkyl or $C_{4-5}$-alkylene. Preferably acetone is suitable as di-lower alkyl ketone. Preferably cyclopentanone and cyclohexanone are suitable as $C_{5-6}$-cycloalkyl ketones.

Where compounds of formula I are to be prepared in which the substituents contained in the subgroup $R^6$, $R^7$, $R^8$, $R^9$, $R^{10}$ and/or $R^{11}$ have meanings other than hydrogen, the point of departure is preferably carbohydrate compounds of Formula IX which contain free hydroxyl groups at least in alpha-position to the aldehyde function. It is beneficial to start with compounds of formula IX wherein $R^7$, $R^8$, $R^9$, $R^{10}$ and $R^{11}$ are hydrogen. The free hydroxyl groups may if desired then be acylated, carbonylated or reacted with a suitable ketone in the above manner.

The compounds of formula VII are novel compounds which are advantageously suitable as intermediate products for the preparation of novel active substances, for example for the preparation of the compounds of formula I, which are antagonistic to tachykinin receptors.

The compounds of formula VII can be prepared by reacting a compound of the general formula VI,

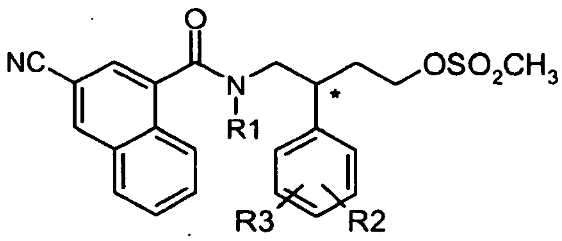

VI

wherein $R^2$ and $R^3$ have the above meanings, with an alkali metal halide MX wherein M stands for an alkali metal, preferably sodium, and wherein X stands for halogen, preferably iodide, and a protected piperazine derivative of the general formula XIX,

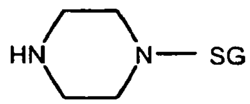

XIX

wherein SG stands for a cleavable protective group, in particular for tert. butoxycarbonyl, to give compounds of the general formula VIa and subsequently cleaving off the protective group SG again in known manner to give compounds of general formula VII. The reaction can be carried out in an organic solvent which is inert under the reaction conditions, such as an aromatic hydrocarbon, in particular toluene, or in a cyclic or open-chain di-lower alkyl ether, in particular THF, or preferably in a mixture of the aforementioned solvents and in the presence of a base. Suitable bases are non-nucleophilic organic nitrogen bases such as tertiary lower alkylamines, for example triethylamine. Suitable reaction temperatures are between 50° and 100°C, preferably approximately 70° to 90°C.

Compounds of formula VI can be prepared by reacting compounds of the general formula V,

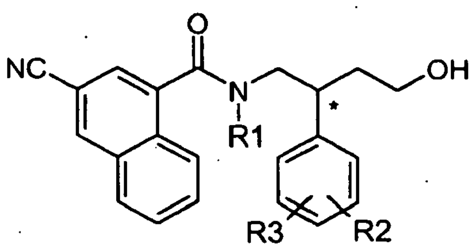

V

wherein $R^1$, $R^2$ and $R^3$ have the above meanings, in known manner with methanesulfonyl chloride. Compounds of formula VI and their stereoisomeric forms are known per se, for example from EP 0 474 561 A1, and can be prepared

according to the processes described in this specification or according to analogous processes.

Compounds of formula XIII can be prepared by reacting compounds of the general formula IV,

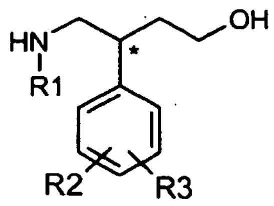

IV

with tertiary butyloxycarbonyl anhydride to give a compound of general formula XX,

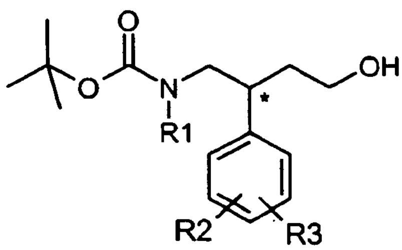

XX

wherein $R^1$, $R^2$ arid $R^3$ have the above meanings. The compounds of the general formula XX are further reacted with methanesulfonyl chloride to give compounds of general formula XXI

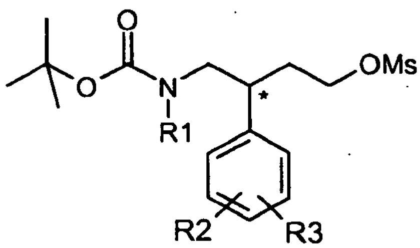

XXI

wherein $R^1$, $R^2$ and $R^3$ have the above meanings. The compounds of general formula XXI are subsequently reacted with an alkalimetal halide MX wherein M stands for an alkali metal, preferably sodium and wherein X stands for halogen, preferably iodide to give compounds of general formula XIII

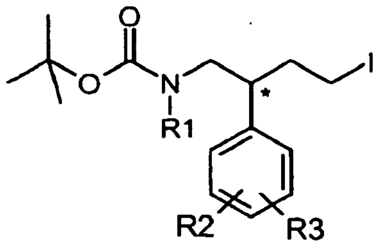

XIII

wherein $R^1$, $R^2$ and $R^3$ have the above meanings.

The compound of the general formula XVI can be prepared by oxidation of compound XX in any manner known from the art, e.g, by dimethylsulfoxide activated with oxalyl chloride (Swem oxidation).

The compounds of formulae VIII, IX and XIX are known per se or can be prepared by the person skilled in the art from known compounds in known manner. Compounds of formula IX which are preferentially used, comprise D-xylose, D-lyxose, D-arabinose, D-threose, D-erythrose and D- and L-glyceraldehyde.

The compounds of formula I may be isolated from the reaction mixture and purified in known manner. Acid addition salts may be converted into the free bases in conventional manner, and these may if desired be converted in

known manner into physiologically compatible acid addition salts. Physiologically compatible salts of compounds of formula I are their conventional salts with inorganic acids, for example sulphuric acid, phosphoric acids or hydrohalic acids, preferably hydrochloric acid, or with organic acids, for example lower aliphatic monocarboxylic, dicarboxylic or tricarboxylic acids such as maleic acid, fumaric acid, lactic acid, tartaric acid, citric acid, or with sulphonic acids, for example lower alkanesulphonic acids such as methanesulphonic acid or trifluoromethanesulphonic acid, or benzenesulphonic acids optionally substituted in the benzene ring by halogen or lower alkyl, such as p-toluenesulphonic acid.

The compounds of formula I contain in the alpha-position to the ring nitrogen atom in the 4-position of the piperazine ring an asymmetrical carbon atom, namely the carbon atom *C bearing the phenyl ring substituted by $R^2$ and $R^3$. Owing to this asymmetrical carbon atom and to the asymmetrical carbon atom bearing the subgroups $R^4$, $R^5$ and $R^6$ and optionally also owing to the asymmetrical carbon atoms contained in the subgroup $R^6$, the compounds of formula I may be present in several stereoisomeric forms. The present invention covers both the mixtures of optical isomers and the isomerically pure compounds of formula I. Preferred are compounds of formula I in which the carbon atom *C bearing the phenyl ring substituted by $R^2$ and $R^3$ is in the S-configuration. If mixtures of optical isomers of the starting compound, for example of the compounds of formula VII or the compounds of formula IX, are used in the synthesis of the compounds of formula I, the compounds of formula I are also obtained in the form of mixtures of optical isomers. Departing from stereochemically uniform forms of the starting compound, stereochemically uniform compounds of formula I can also be obtained. The stereochemically uniform compounds of formula I can be obtained from the mixtures of optical isomers in known manner, for example by chromatographic separation on chiral separating materials or by reaction with suitable optically active acids, for example tartaric acid or 10-camphorsulphonic acid, and subsequent separation into their optically active antipodes by fractional crystallisation of the diastereomeric salts obtained.

The compounds of formula I and their acid addition salts have properties which are antagonistic to tachykinin receptors and are therefore suitable for the treatment of pathological conditions in larger mammals, particularly humans, in which tachykinins are involved as transfer agents. The group of compounds according to the invention is distinguished by a particularly beneficial action profile which is characterised by a high selective affinity to $NK_1$- and $NK_2$ receptors. Furthermore, the group of compounds according to the invention is distinguished by good compatibility even over prolonged periods of administration, and by comparatively good oral availability. Owing to their action profile, the compounds of formula I are suitable in particular for inhibiting processes in which tachykinins, such as neurokinin A, which bind to $NK_1$ and to $NK_2$ receptors are involved. Owing to the action which is advantageously directed at the peripheral region, the compounds of formula I are suitable in particular for the treatment and/or prophylaxis of respiratory diseases, in particular asthma, bronchitis, cough, and rhinitis; skin diseases, in particular inflammatory skin reactions, allergic skin reactions, and psoriasis; arthropathy diseases, in particular arthtitis, vasculitides and systemic lupus erythematosus; functional or inflammatory disorders in the gastrointestinal tract, in particular pseudomembranous colitis and diarrhoe; bleb diseases such as cystitis and interstitial cystitis; and migraine, of larger mammals, particularly humans, of both sexes, also including diseases which involve increased sensitivity to pain and/or impaired stool passage in the colon region. The functional disorders in the gastrointestinal tract which can be treated by the compounds according to the invention include in particular the disorders of the lower intestinal tracts known under the name "irritable bowel syndrome" (= IBS). Typical symptoms for the diagnosis of IBS are described, for example, in W.G. Thompson et al., Gastroenterology International 2 (1989) 92-95 or in W.G. Thompson et al., GUT 45/II (1999) II43-II47, and are generally known among experts by the term "Rome Criteria". The essential symptoms of IBS accordingly include pains in the lower abdomen, which appear to be due to hypersensitivity of the visceral afferent nervous system, and anomalies in bowel movement, such as constipation, diarrhoea or alternating constipation and diarrhoea. Further inflammatory disorders in the gastrointestinal tract which can be beneficially influenced by the group of compounds according to the invention are for example the inflammatory disorders in the small intestine and large intestine regions usually covered by the term "inflammatory bowel disease" (= IBD), for example ulcerative colitis or Crohn's disease. Owing to their action mechanism, the compounds according to the invention furthermore appear suitable for the treatment of other disorders in which tachykinins and in particular neurokinin A are involved as transfer agents. These disorders include for example neurogenic inflammations, inflammatory joint diseases such as rheumatic arthritis, asthmatic complaints, allergic disorders, disorders of immune regulation, bladder inflammation or also functional dyspepsia.

Description of the pharmacological test methods

The example numbers given for the compounds of formula I used as test substances in the pharmacological tests given below relate to the preparation examples described below.

1. Determination of the binding power of the test substances to $NK_1$ receptors in vitro.

In The Netherlands, the affinity of the test substances to human $NK_1$ receptors was measured in vitro. The inhibition of the binding of the physiological neurokinin (Substance P) to neurokinin-1 receptors was determined in The

Netherlands.

The receptor binding studies were performed with [$^3$H]-Substance P as ligand. For the binding test, different samples of a membrane preparation of CHO cells (= egg cells of the Chinese hamster, Chinese hamster oocytes), which express the human $NK_1$ receptor ("Accession Number" of the associated nucleic acid sequence = M74290; "Accession Number" of the associated protein sequence = P25103; cf. Takeda, Y.; Chou, K. B., Takeda, J.; Sachais, B.S. and Krause, J. E; Biochemical and Biophysical Research Communications, 179(3) (1991) 1232 - 1240), were incubated with a solution of the marked ligand, with the incubation mixtures containing no test substance or additions of different concentrations of test substance. Then, separation of bound and free ligands was performed in each of the samples with the aid of glass-fibre filtration. The fraction remaining in the filter was washed several times with buffer solution and then the radioactivity of the fraction remaining in the filter was measured using a beta scintillation counter.

For the compounds of Examples 1 to 14, that concentration which effects half maximum displacement of the bound ligand was determined as $IC_{50}$ of the respective test substance. From this, the corresponding inhibition constant ($K_i$ value) of the test substance was calculated, and was stated as the negative common logarithm of the $K_i$ value (= $pK_i$ value). The $pK_i$ value is a measurement of the affinity of the test substances to human $NK_1$ receptors. In this test model, the test substances set forth in Table 1 below exhibited the given $pK_i$ values:

Table 1: Affinity of the test substances to human $NK_1$ receptors

| Compound No. | pKi ($NK_1$) |
|---|---|
| 1 | 8,4 |
| 2 | 8,5 |
| 3 | 8,8 |
| 4 | 8,6 |
| 5 | 8,9 |
| 6 | 8,3 |
| 7 | 8,3 |
| 8 | 8,1 |
| 9 | 8,0 |
| 10 | 8,0 |
| 11 | 8,1 |
| 12 | 8,0 |
| 13 | 8,1 |
| 14 | 8,3 |
| 16 | 9,0 |
| 17 | 7,5 |
| 18 | 8,5 |
| 21 | 8,7 |
| 22 | 8,4 |
| 24 | 8,0 |
| 25 | 8,1 |
| 26 | 8,1 |
| 27 | 8,2 |
| 28 | 7,9 |

All the aforementioned test substances exhibited pKi values of at least 7,0 in this test model. The compounds of Examples 1 to 14 exhibited pKi values of at least 7,9.

2. Determination of the binding power of the test substances to $NK_2$ receptors in vitro

The affinity of the test substances to human $NK_2$ receptors was measured in vitro. The ability of the test substances to displace the selective $NK_2$ receptor antagonist SR 48968 (= saredutant) used as reference ligand from its corresponding bond was determined.

The receptor binding studies were carried out with radioactively marked [$^3$H]-SR 48968 (from Amersham) as ligand. For the binding test, different samples of a membrane preparation of CHO cells (= egg cells of the Chinese hamster, Chinese hamster oocytes), which express the human $NK_2$ receptor (for preparation, see N.P. Gerard et al., Journal of Biological Chemistry 265/33 (1990) 20455-20462), were incubated for 90 minutes (= min.) with a solution of the marked ligand, with the incubation mixtures containing no test substance or additions of different concentrations of test substance. Then in each case the membrane-bound ligands in the samples were separated from free ligands by filtration. The fraction remaining in the filter was washed several times with buffer solution, before its radioactivity was measured using a liquid scintillation counter. That concentration which effects half-maximum displacement of the bound reference ligand was determined as $IC_{50}$ of the respective test substance. The inhibition constant (Ki value) of the test substance was calculated from the respective $IC_{50}$ value, and was stated as the negative logarithmised value thereof (pKi).

For the compounds of Examples 1 to 14, the affinity to human $NK_2$ receptors was determined in each case by at least three measurements of the test substances in concentration series of $10^{-6}$ to $10^{-10}$ mol/l. If several measurements were performed, the average thereof was listed each time. The $pK_i$ value is a measurement of the affinity of the test substances to human $NK_2$ receptors. In this test model, the test substances set forth in table 2 below exhibited the given $pK_i$ values:

Table 2: Affinity of the test substances to human $NK_2$ receptors

| Compound No. | pKi (NK2) |
|---|---|
| 1 | 8,1 |
| 2 | 7,1 |
| 3 | 7,2 |
| 4 | 6,9 |
| 5 | 7,3 |
| 6 | 7,4 |
| 7 | 7,4 |
| 8 | 7,5 |
| 9 | 7,2 |
| 10 | 7,0 |
| 11 | 7,6 |
| 12 | 7,0 |
| 13 | 7,7 |
| 14 | 7,9 |
| 16 | 6,1 |
| 17 | 5,5 |
| 18 | 6,8 |
| 21 | 6,4 |
| 22 | 6,0 |
| 24 | 7,5 |
| 25 | 7,4 |
| 26 | 7,0 |
| 27 | 7,6 |

(continued)

| Compound No. | pKi (NK2) |
|---|---|
| 28 | 7,3 |

All the aforementioned test substances exhibited pKi values of at least 7,0 in this test model. The compounds of Examples 1 to 14 exhibited pKi values of at least 6,9.

3. Determination of the functional $NK_1$ antagonism of the test substances on isolated guinea pig tissue in vitro

In The Netherlands, the action antagonistic to $NK_1$ receptors of the test substances was measured in vitro on isolated ring preparations, kept in an oxygenated nutrient solution, of the aortas of Pirbright-White guinea pigs. The inhibition by the test substances of the relaxation of tone of the aorta preparations, caused after stimulation with the $NK_1$ agonist Substance P, was determined.

In order to measure the contraction of the vessel muscles, the preparations were fixed to a hook, joined by a thread to a force measuring apparatus and the contractions were recorded in each case on a plotter. The aorta preparations were tonicised with phenylephrine. Then before and after the administration of the test substance the $NK_1$ receptors of the preparations were stimulated with 0.01 μmol Substance P, which caused relaxation of the tone. The relaxations before and after the administration of the test substance were quantified in percent. The effective concentration of the half maximum inhibition of the relaxation of the tone (= $EC_{50}$) was calculated. The negative common logarithm of the $EC_{50}$ value (= $pEC_{50}$) was given as characteristic variable. The $pEC_{50}$ value is a measurement of the functional effectiveness of the test substances on $NK_1$ receptors. In this test model, the test substances set forth in Table 3 below exhibited the given $pEC_{50}$ values:

Table 3: Functional $NK_1$ antagonism of the test substances on isolated guinea pig tissue

| Example No. | $pEC_{50}$ |
|---|---|
| 1 | 9,4 |
| 2 | 9,5 |
| 5 | 10,0 |
| 6 | 9,4 |
| 11 | 8,9 |

4. Determination of the functional antagonism of the test substances on isolated guinea pig tissue in vitro

The $NK_2$ receptor-antagonising action of the test substances was determined on isolated gall-bladder preparations from Pirbright-White guinea pigs, held in an oxygen-saturated nutrient solution. To this end, the preparations were fastened on one hand in the nutrient solution to organ holders and on the other hand on a force meter by a thread.

In this test the $NK_2$ receptors present in the gall-bladder preparations were stimulated with the natural $NK_2$ receptor agonist neurokinin A (= NKA; 0.1 μmol/l) and the contractions of the preparations caused thereby were measured as contractility in mN (= preliminary value) measured. Then NKA was rinsed out of the preparations with NKA-free solution and the test substances were added in a concentration of $10^{-7}$ mol/l. After two hours' incubation of the preparations with the test substances, the contractions of the preparations then still caused by renewed NKA addition were again measured and the results were given as percentages, relative to the contractions initially measured, caused solely by NKA addition. The concentration of the test substances was increased iteratively in the subsequent experiments as a function of the result in logarithmic whole or half steps, until at least one concentration above or below 50% inhibition of contraction was determined (up to at most $10^{-5}$ mol/l). For each concentration, the average value of inhibition of contraction was calculated from 2 to 4 preparations. In each case, the concentration of half-maximum inhibition ($IC_{50}$) per test substance was calculated as characteristic variable. In each case the logarithmised value of the $IC_{50}$ per test substance is given as $pIC_{50}$ in (mol/l]. In this test model, the test substances set forth in Table 4 below exhibited the $pIC_{50}$ values given below.

Table 4: Functional $NK_2$ antagonism of the test substances on isolated guinea pig tissue

| Example No. | $pIC_{50}$ |
|---|---|
| 1 | 8,7 |
| 2 | 7,7 |
| 5 | 7,5 |
| 6 | 7,4 |
| 11 | 7,8 |

5. Functional cellular tests of the $NK_1$- and $NK_2$-antagonistic

Functional cellular tests of the antagonistic effect of the compounds of the present invention on the human tachykinin receptors were performed in CHO cells expressing the recombinant human $NK_1$ or NK2 receptor. In these tests the inhibition of ligand induced increase in mobilization of intracellular calcium and inhibition of ligand induced phosphorylation of MAPK were determined, which can be used as a measure of functional activity of tachykinin-antagonists. Additionally, the antagonistic properties of reference compounds on the different tachykinin receptors were characterized for comparison.

The effects of test compounds were assessed using Chinese hamster ovary (CHO) fibroblast cells, stably expressing cloned human $NK_1$ or NK2 receptors. The NK receptor is coupled to $G_q$. The activation of the $G_q$ protein by ligand binding to the receptor leads to a mobilization of intracellular calcium and phosphorylation of MAPK. Both systems were used to determine functional effects of the test compounds.

*$Ca^{2+}$ measurements using FLIPR for $NK_1$ and $NK_2$ activity*

For tests, cells were seeded 24 hours prior to the experiment into black 96-well microplates. The cell density was $2.2\times10^4$ cells/well. All steps were done under sterile conditions. In order to observe changes in intracellular calcium levels, cells were loaded with a calcium-sensitive dye. This dye (FLUO-4, from Molecular Probes) excites at 488 nm, and emits in the 500 nm to 560 nm range, only if a complex with calcium is formed. For the dye loading the growth-medium was aspirated out of the well without disturbing the confluent cell layer and 100 μl loading medium (HBSS, 4 μM FLUO-4, 0.005% (w/v) pluronic acid, 2.5mM probenecid, 20 mM HEPES, pH 7.4) was dispensed into each well using an automatic pipettor system (Multidrop, Labsystems). Pluronic acid was added to increase dye solubility and dye uptake into the cells, whereas probenecid, an anion exchange inhibitor, was added to the loading medium to increase dye retention in the cells. The cells were incubated in a 5% $CO_2$ incubator at 37°C for 40 minutes. After dye loading, the cells were washed three times with wash-buffer (HBSS, 2.5 mM probenecid, 20 mM HEPES, pH 7.4) to reduce basal fluorescence. In the last washing step the buffer was aspirated and replaced with 100 μl washing buffer. For the antagonism screening mode 50 μl of the compound (final concentration ranges from 10 μM to 1.4 nM) were applied 7 min prior to addition of substance P (final concentration:$10^{-8}$ M; $NK_1$ agonist) or NKA (final concentration: $10^{-7}$ M; NK2 agonist). The FLIPR setup parameters were set to 0.4 sec exposure length, filter 1, 50 μl fluid addition, pipettor height at 125 μl, dispense speed 40 μl/sec without mixing. Maximal fluorescence changes were obtained using the statistic function of the FLIPR software, and data plotted using GraphPad Prism 4. All points were expressed as a percentage inhibition of the control agonist. $IC_{50}$ values were determined using sigmoidal dose-response curve fitting. Antagonist potencies ($pA_2$) values were calculated using equation:

$$pA_2 = -\log (IC_{50}/(1 + [L] / EC_{50})),$$

in which the $IC_{50}$ of the test compound was obtained from concentration-effect relationships, [L] is the concentration of the agonist (substance P for $NK_1$ test, NKA for NK2 test), and the $EC_{50}$ is the potency of the agonist at the respective human cloned NK receptor ($EC_{50}$ substance P: $10^{-9.6}$ M; $EC_{50}$ NKA: $10^{-8.8}$ M). The results are summarized in table 5:

Table 5: $pA_2$ data for $NK_1$ and NK2:

| Compound No. | $pA_2$ ($NK_1$) | $pA_2$ ($NK_2$) |
|---|---|---|
| 1 | 8,9 | 8,0 |
| 7 | 8,8 | 8,1 |

(continued)

| Compound No. | $pA_2$ ($NK_1$) | $pA_2$ ($NK_2$) |
|---|---|---|
| 8 | 9,2 | 8,9 |
| 9 | 8,5 | 8, |
| 13 | 8,3 | 7,7 |
| 14 | 9,4 | 8,5 |
| 16 | 9,3 | 9,1 |
| 17 | 7,7 | 7,7 |
| 18 | 8,2 | 8,6 |
| 21 | 9,1 | 9,5 |
| 22 | 8,1 | 8,5 |
| 24 | 8,1 | 7,8 |
| 25 | 8,5 | 9,0 |
| 26 | 8,0 | 7,4 |
| 27 | 9,2 | 8,1 |
| 28 | 8,7 | 7,6 |

6. Determination of the NK-1- and NK-2-receptor-antagonistic effectiveness of the test substances in vivo

The NK-1- and NK-2-antagonistic activities of the test substances were investigated in anaesthetised guinea pigs in each case after intravenous (= i.v.) and oral (= p.o.) administration in vivo. With the present test model it is possible to detect both NK-2-antagonistic effects in three different organ systems (respiratory tracts, colon and circulation) and NK-1-antagonistic effects (rapid drop in blood pressure) in an animal simultaneously.

Pirbright-White guinea pigs of a body weight of 500-700 g were anaesthetised with ketamine/xylazine (67/13 mg/kg subcutaneously, initial dose, further doses administered as required). The animals were provided with an intravenous catheter in order to administer the substance and an intra-arterial catheter to measure the blood pressure. The animals were artificially ventilated via a tracheal cannula and the respiratory pressure was recorded by means of a pressure transducer. A balloon was introduced into the distal colon of the animals for manometric recording of colon motility by means of a pressure transducer. Blood pressure, heart rate, respiratory pressure and colonic pressure were measured continuously for each animal and plotted on a recorder and by means of a digital data-processing system. Neurokinin A (= NKA; 200 pmol/animal) was administered i.v. as a bolus as a test stimulus to stimulate the NK-1-and the NK-2 receptors. An addition of NKA of this type results in a great increase in respiratory pressure (bronchoconstriction) and colonic pressure, and in a biphasic drop in blood pressure. The first phase of hypotension (= phase of maximum hypotension within the first minute after administration of NKA) is mediated via NK-1 receptors, since they can be blocked completely by specific NK-1 receptor antagonists. The second phase of delayed hypotension (= phase of maximum hypotension after 2-5 min.) on the other hand is mediated via NK-2 receptors, since they can be blocked by specific NK-2 receptor antagonists. The doses of the test substances are given as $ED_{50}$ values which each result in a response to the NKA test stimulus which is reduced to 50% of the initial value, as characteristic variables for the individual measurement parameters bronchoconstriction, colonic pressure and change in blood pressure mediated by NK-1 or NK-2.

Table 6: NK-1- and NK-2-receptor-antagonistic effective-ness of the test substances of Formula I on guinea pigs in vivo after intravenous administration

| | $ED_{50}$ iv [$\mu$mol/kg] after 1 min (cumulative) | | | |
|---|---|---|---|---|
| Structure | NK1 (early hypotension) | NK2 (late hypotension) | NK2 (broncho-constriction) | NK2 (colonic motility) |
| 1 | 0.077 | 0.643 | 0.321 | 0.027 |
| 2 | <0.010 | 0.106 | <0.010 | 0.085 |
| 6 | 0.071 | 0.282 | 0.595 | 0.050 |

(continued)

| | $ED_{50}$ iv [μmol/kg] after 1 min (cumulative) | | | |
|---|---|---|---|---|
| Structure | NK1 (early hypotension) | NK2 (late hypotension) | NK2 (broncho-constriction) | NK2 (colonic motility) |
| 7 | 0.052 | 0.338 | 0.207 | 0.309 |
| 8 | 0.070 | 0.803 | 1.126 | 0.135 |
| 9 | 0.099 | 1.044 | 0.943 | 0.287 |
| 10 | 0.041 | 0.040 | 0.045 | 0.109 |
| 11 | 0.093 | 0.145 | 0.130 | 0.132 |
| 23 | 0.029 | 1.165 | 0.725 | 0.961 |

The antagonistic effects of the test substances were first investigated in cumulative form, the time of the NKA test stimulus being 1 min after the administration of the respective doses of the test substances had ended. These $ED_{50}$ values obtained from cumulative dose effect curves are plotted in table 6.

The measured values plotted in table 6 above show, inter alia, that the substances of structures 1, 2 and 6 to 11 after cumulative administration i.v. (detection of the antagonism 1 min. after the administration of test substance had ended) caused a marked NK-1-receptor-antagonistic activity on the early hypotension as well as NK-2-receptor-antagonistic activity of colon motility, late drop in blood pressure and respiratory resistance.

In order additionally to detect the variation over time of the antagonistic effects of the test substances, the action of the NKA test stimulus was determined at different times (1, 30, 60, 90, 120, 150 and 180 min.) after oral administration of the test substances. The antagonistic effects of the test substances were then determined as "area under the curve" ("AUC") over the investigation period after administration of the test substances (1 - 180 min after administration) and the $ED_{50}$ values after oral administration obtained therefrom were plotted in table 7.

Table 7: NK-1- and NK-2-receptor-antagonistic effective-ness of the test substances of Formula I on guinea pigs in vivo after oral administration

| | $ED_{50}$ $AUC_{1\text{-}180\ min}$ oral [μmol/kg] | | | |
|---|---|---|---|---|
| Structure | NK1 (early hypotension) | NK2 (late hypotension) | NK2 (broncho-constriction) | NK2 (colonic motility) |
| 1 | 22.9 | 3.1 | 3.1 | 5.5 |
| 2 | 15.2 | 14.8 | 11.7 | 6.0 |

The compounds according to the invention, in particular the substance of structures 1 and 2 as shown in table 7, are furthermore active orally on the NK2 as well as NK1 receptor antagonists.

The compounds of formula I may be administered in conventional pharmaceutical preparations. The doses to be used may vary individually and will naturally vary according to the type of condition to be treated and the substance used. In general, however, medicinal forms with an active substance content of 0.2 to 200 mg, in particular 1 to 50 mg, active substance per individual dose are suitable for administration to humans and larger mammals. The compounds may be contained according to the invention, together with conventional pharmaceutical auxiliaries and/or carriers, in solid or liquid pharmaceutical preparations. Examples of solid preparations are preparations which can be administered orally, such as tablets, coated tablets, capsules, powders or granules, or alternatively suppositories. These preparations may contain conventional pharmaceutical inorganic and/or organic carriers, such as talcum, lactose or starch, in addition to conventional pharmaceutical auxiliaries, for example lubricants or tablet disintegrating agents. Liquid preparations such as suspensions or emulsions of the active substances may contain the usual diluents such as water, oils and/or suspension agents such as polyethylene glycols and the like. Other auxiliaries may additionally be added, such as preservatives, taste correctives and the like.

The active substances may be mixed and formulated with the pharmaceutical auxiliaries and/or carriers in known manner. For the production of solid medicament forms, the active substances may for example be mixed with the auxiliaries and/or carriers in conventional manner and may be wet or dry granulated. The granules or powder can be poured directly into capsules or be pressed into tablet cores in conventional manner. These may be coated in known manner if desired.

The following examples are intended to explain the invention further, without limiting its scope.

Example 1:

3-cyano-*N*-[(2*S*)-2-(3,4-dichlorophenyl)-4-{4-[(1*R*,2*S*)-1-(2-furyl)-2,3-dihydroxypropyl]-piperazin-1-yl}butyl]-*N*-methyl-1-naphthamide (process 1)

A) 58.0 g 3-cyano-naphthalene-1-carboxylic acid (formula II) was suspended in 600 ml of dichloromethane. 2 ml of DMF were added successively under stirring. To this initial suspension, 35 ml of oxalyl dichloride in 65 ml dichloromethane were added slowly. The mixture was stirred for 4 hours at 30 °C to 40 °C. The obtained solution was concentrated to dryness and 67 g of 3-cyano-naphthalene-1-carbonyl chloride (formula III) was isolated, stored in a refrigerator and used without further purification.

B) 20 g of 3*S*-(3,4-Dichloro-phenyl)-4-methylamino-butan-1-ol (formula IV) were suspended in 200 ml of THF under stirring at room temperature. 12 g of KOH dissolved in 100 ml of water were added leading to a solution. 17.2 g of 3-cyano-naphthalene-1-carbonyl chloride (formula III, from reaction step A)) were added and stirred for 3 hours. The organic solvents were eliminated and the remaining mixture was supplemented with ethyl acetate and methyl-tert.-butylether. The water phase was eliminated while the organic phase was washed four times with 50 ml of water and dried over sodium sulfate. The organic phase was concentrated to dryness, providing 31.8 g of a yellowish solid (3-cyano-naphthalene-1-carboxylic acid [2*S*-(3,4-dichloro-phenyl)-4-hydroxybutyl]-methyl-amide; formula V) which was used without further purification.

C) 5.8 g of 3-cyano-naphthalene-1-carboxylic acid [2S-(3,4-dichloro-phenyl)-4-hydroxy-butyl]-methyl-amide (formula V, from reaction step B)) were dissolved in 100 ml dichloromethane. 2.2 ml of triethylamine and 1.16 ml of methanesulfonyl chloride were added at room temperature. The reaction mixture was stirred for 5 hours and left for 2.5 days. Water was added and the organic part was dried over sodium sulfate and concentrated to dryness. 6.65 g of a foamy product (3-cyano-naphthalene-1-carboxylic acid [(2*S*)-2-(3,4-dichloro-phenyl)-4-methylsulfonyl-butyl]-methyl-amide, formula VI) were isolated and used without further purification.

D) 6.65 g of 3-Cyano-naphthalene-1-carboxylic acid [2S-(3,4-dichloro-phenyl)-4-methylsulfonyl-butyl]-methyl-amide (formula VI, from reaction step C)) were dissolved in 150 ml of acetonitril. 1.84 g of potassium iodide, 2.44 g N-tert. butoxycarbonyl-piperazine (formula XIX) and 2.2 ml triethylamine were added and the mixtures was heated to reflux for three hours. After cooling down to room temperature, the reaction mixture was left over night before adding water and ethyl acetate. The organic phase was washed with water and a saturated solution of sodium bicarbonate. Drying of the organic phase over sodium sulphate and evaporation of the solvent in a vacuum yielded 7.8 g 3-cyano-naphthalene-1-carboxylic acid {2*S*-(3,4-dichloro-phenyl)-4-[4-(2,2-dimethyl-propionyl)-piperazin-1-yl]-butyl}-methyl-amide (formula VIa), which was used directly for further reactions without further purification.

E) 6.6 g 3-cyano-naphthalene-1-carboxylic acid {2*S*-(3,4-dichloro-phenyl)-4-[4-(2,2-dimethyl-propionyl)-piperazin-1-yl]-butyl}-methyl-amide (formula VIa, from reaction step D)) were dissolved in 150 ml ethanol and 20 ml of 5N HCl were added. The mixtures was stirred for 2 days and neutralized with sodium carbonate. Ethanol was distilled off and the product was extracted with ethyl acetate and water. The ethyl acetate layer was dried over sodium sulfate and concentrate to dryness, providing 5.2 g of amorphous 3-cyano-naphthalene-1-carboxylic acid [2*S*-(3,4-dichloro-phenyl)-4-piperazin-1-yl-butyl]-amide (formula VII).

F) 3.0 g of 3-cyano-naphthalene-1-carboxylic acid [2*S*-(3,4-dichloro-phenyl)-4-piperazin-1-yl-butyl]-amide (formula VII, from reaction step E)), 806 mg of 2-furanboronic acid (formula VIII) and 945 mg of 80% pure (D)-glyceraldehyde (formula IX) in ethanol were heated to reflux for 5 hours. The mixture was kept at room temperature over night before distilling off the organic solvent. The product obtained was purified by column chromatography (ethyl acetate till ethanol) to deliver 1.3 g of pure crystalline 3-cyano-*N*-[(2*S*)-2-(3,4-dichlorophenyl)-4-{4-[(1*R*,2*S*)-1-(2-furyl)-2,3-dihydroxypropyl]piperazin-1-yl}butyl]-*N*-methyl-1-naphthamide (formula I).
$[\alpha]_D^{20}$ = -27,6° (c = 1, methanol).
MS-data (ES+): M+ bei m/z = 635
Melting point = 140-142 °C

Example 2:

3-cyano-*N*-[(2*S*)-2-(3,4-dichlorophenyl)-4-{4-[(1*R*,2*S*)-1-(2-furyl)-2,3-dihydroxypropyl]-piperazin-1-yl}butyl]-*N*-methyl-1-naphthamide (process 2)

A) 23.0 g of tert. butoxycarbonyl-piperazine (formula X) were dissolved in 600 ml ethanol under nitrogen at 30 °C. 25 g of 2-furanboronic acid (formula VIII) and 22.0 g of 80% pure (D)-glyceraldehyde (formula IX) in 400 ml ethanol were heated to reflux for 7 hours. The mixture was cooled down to room temperature and concentrated to dryness. The residue was dissolved in 200 ml ethyl acetate and 50 ml methyl-tert.-butylether and successively washed with a solution of 20 g of KOH in 200 ml water. The residue was further washed with six portions of 150 ml water before drying over sodium sulfate. After evaporation of the solvent, 54.7 g of 4-((2*S*)-1-furan-2-yl-2-hydroxy-propyl)-piperazine-1-carboxylic acid tert-butyl ester (formula XI) was obtained.
$[\alpha]_D^{20}$ = +34.6° (c = 1, methanol).
Melting point: 92 to 93°C.

B) 16.3 g of 4-((2*S*)1-furan-2-yl-2-hydroxy-propyl)-piperazine-1-carboxylic acid tert-butyl ester (formula XI, from reaction step A)) were dissolved in 50 ml methyl-tert.-butylether under stirring. 160 ml of 5 N HCl were added to give a solution and a strong gas evolution. After stirring for 24 hours, the solution was poured in 200 ml methyl-tert.-butylether and further stirred. A solid precipitate which was filtered and washed with 20 ml of methyl-tert.-butylether. After drying under vacuum at 60 °C 13.5 g of (2*S*)-1-furan-2-yl-1-piperazin-1-yl-butan-2-ol dihydrochlorid (formula XII) were isolated.
Specific rotation: +14.0° (c = 1, methanol).

C) 28.4 g of sodium hydrogen carbonate in 150 ml water were added to a suspension of 25 g of 2*S*-(3,4-Dichloro-phenyl)-4-methylamino-butan-1-ol (formula IV) hydrochlorid in 500 ml of THF under stirring. A solution of 21.1 g of tertiary butyloxycarbonyl anhydride in 200 ml THF was added and the combined reaction mixture was stirred for four hours. The THF was distilled off under vacuum and 500 ml of ethyl acetate was added to the residue. 100 ml of a saturated solution of sodium hydroxyl carbonate was added, followed by the addition of 300 ml water. The organic phase was removed and dried over sodium sulfate. The organic solvent was removed to deliver an oily material which was purified in column chromatographie on Silica gel to give 29.5 g of [(2*S*)-2--(3,4-dichloro-phenyl)-4-hydroxy-butyl]-methyl-carbamic acid tert-butyl ester (formula XX) as a colorless oily pure material which slowly crystallizes. 20 g of [(2*S*)-2--(3,4-dichloro-phenyl)-4-hydroxy-butyl]-methyl-carbamic acid tert-butyl ester (formula XX) were dissolved under stirring in 150 ml of dichloromethane at room temperature under nitrogen to which 6 ml of triethylamine and 3 ml methanesulfonyl chloride were added dropwise. The solution was stirred for three days at room temperature and finally concentrated. 200 ml of ethyl acetate and toluene were added and the organic layer was washed with 100 ml water, with a saturated solution of sodium hydrogen carbonate until pH 8 to 9 was reached and finally washed with 100 ml water. The organic phase was dried over sodium sulfate and evaporated to dryness to give 13.3 g of the corresponding mesylate (formulae XXI). 13.3 g of the mesylate were dissolved under stirring in 200 ml of acetone at room temperature under nitrogen and 22 g of sodium iodide were added. The mixture was stirred until completion of the reaction (three days). Acetone was distilled off and 200 ml ethyl acetate were added as well as 33 g of sodium thiosulfate pentahydrate dissolved in 200 ml water. The aqueous phase was then separated and the organic phase was successively washed with 100 ml of an saturated solution of sodium hydrogen carbonate followed by three times of 100 ml water. The organic phase was dried over sodium sulfate and concentrated to dryness to give 15.1 g of [(2*S*)-2-(3,4-dichloro-phenyl)-4-iodo-butyl]-methyl-carbamic acid tert-butyl ester (formulae XIII).

D) A 10 ml solution of 200 mg of [(2*S*)-2-(3,4-dichloro-phenyl)-4-iodo-butyl]-methyl-carbamic acid tert-butyl ester (formulae XIII, from reaction step C)) in 10 ml THF were added to a suspension of 450 mg of (2*S*)-1-furan-2-yl-1-piperazin-1-yl-butan-2-ol dihydrochlorid (formula XII, from reactions step B)) and 1ml of triethylamine in 50 ml THF under stirring at room temperature. About 100 mg of $Na_2CO_3$ were added and the mixture was boiled to reflux for 15 hours. After cooling to room temperature, the suspension was concentrated in vacuum and the residue was dissolved in 50 ml of ethyl acetate and 200 mg of KOH in 10 ml of water. The organic phase was washed 4 times with 20 ml of water, dried on sodium sulfate and evaporated to dryness to deliver 203 mg of a yellowish compound identified in LC-MS as the expected amine XIV.

E) 3 ml of HCl (5N) in isopropanol were added to 460 mg of the amine XIV from reaction step D) dissolved at 30°C

in 2 ml methylene chloride under stirring at room temperature. After 1 hour, a precipitate appeared and 50 ml of methyl-tert.-butylether were added and the mixture was stirred for 15 hours. The white solid was isolated by filtration; washed 3 times with 10 ml methyl-tert.-butylether and dried in vacuum at 80°C. The remaining amount of methyl-tert.-butylether was eliminated by dissolving the compound in methanol and distilling off the solvents to deliver 407 mg (yield: 85%) of a foam used without further purification.

F) A suspension of 168 mg 3-cyano-naphthalene-1-carbonyl chloride (formula III, from reaction step A) of process 1) in 20 ml of methylene chloride were added dropwise to 367 mg of the amine from reaction step E) dissolved in 10 ml of THF, 10 ml of water and 200 mg of KOH. After stirring for 15 hour at room temperature, the reaction mixture was concentrated in vacuum and redissolved in a mixture of 30 ml of ethyl acetate and 30 ml methyl-tert.-butylether. The organic phase was washed 4 times with 20 ml of water, dried on sodium sulfate and concentrated to dryness under vacuum to deliver 420 mg of 3-cyano-*N*-[(2*S*)-2-(3,4-dichlorophenyl)-4-{4-[(1*R*,2*S*)-1-(2-furyl)-2,3-dihydroxypropyl]-pipera-zin-1-yl}butyl]-*N*-methyl-1-naphthamide (formula I) identified in LC-MS and NMR.
$[\alpha]_D^{20}$ = -27,6° (c = 1, methanol).
MS-data (ES+): M+ bei m/z = 635
Melting point = 140-142 °C

Example 3:

3-cyano-*N*-[(2*S*)-2-(3,4-dichlorophenyl)-4-{4-[(1*R*,2*S*)-1-(2-furyl)-2,3-dihydroxypropyl]-piperazin-1-yl}butyl]-*N*-methyl-1-naphthamide (modified process 2)

A) 12.2 g DMSO in 100 ml dichloromethane are added dropwise to 7.3 g oxalyl chloride in 100 ml dichloromethane unter nitrogen at - 70 °C unter stirring. The resulting mixture was stirred for another 15 minutes before 20 g of [2*S*-(3,4-dichloro-phenyl)-4-hydroxy-butyl]-methyl-carbamic acid tert-butyl ester in 200 ml dichloromethane were added. The mixture was stirred at - 70°C for one hour before 40.3 ml of triethylamine in 50 ml dichloromethane were added dropwise. The solution was stirred at - 70°C for 15 minutes and then allowed to warm up to room temperature. The solvent was removed and the residue was dissolved in 300 ml of toluene and 200 ml of ethyl acetate. The resulting solution was washed six times with 200 ml of a saturated solution of NaCl in water, dried over sodium sulfate and concentrated to dryness to deliver 19.7 g of [(2*S*)-2-(3,4-dichloro-phenyl)-4-oxo-butyl]-methyl-carbamic acid tert-butyl ester aldehyde (formula XVI).

B) A mixture of 300 mg of (2*S*)-1-furan-2-yl-1-piperazin-1-yl-butan-2-ol dihydrochlorid (formula XII, from reaction step B) of Process 2), 200 mg of sodium acetate in 50 ml THF and 100 µl of acetic acid were added to a suspension of 280 mg of [(2*S*)-2-(3,4-dichloro-phenyl)-4-oxo-butyl]-methyl-carbamic acid tert-butyl ester aldehyde (formula XVI, from reaction step A)) in 20 ml THF. The resulting mixture was stirred for 5 hours at room temperature. Subsequently, 240 mg of sodium triacetoxyboro-hydride were added and the suspension was stirred for 15 hours at room temperature. The suspension was then concentrated in vacuum, and the residue was dissolved in 30 ml of ethyl acetate, 30 ml of methyl-tert.-butylether and 600 mg of KOH in water. The organic phase was washed 4 times with 20 ml of water. The organic solvents were dried over sodium sulfate and distilled off to give 463 mg of a glassy compound used without further purification in accordance with process steps E) and F) of process 2.

Example 4:

3-cyano-*N*-[(2*S*)-2-(3,4-dichlorophenyl)-4-{4-[(1*R*,2*S*)-1-(2-furyl)-2,3-dihydroxypropyl]-piperazin-1-yl}butyl]-N-methyl-1-naphthamide (process 3)

A) 58.0 g 3-cyano-naphthalene-1-carboxylic acid (formula II) was suspended in 600 ml of dichloromethane. 2 ml of DMF were added successively under stirring. To this initial suspension, 35 ml of oxalyl dichloride in 65 ml dichloromethane were added slowly. The mixture was stirred for 4 hours at 30°C to 40 °C. The obtained solution was concentrated to dryness and 67 g of 3-cyano-naphthalene-1-carbonyl chloride (formula III) was isolated, stored in a refrigerator and used without further purification.

B) 20 g of 3S-(3,4-Dichloro-phenyl)-4-methylamino-butan-1-ol (formula IV) were suspended in 200 ml of THF under

stirring at room temperature. 12 g of KOH dissolved in 100 ml of water were added leading to a solution. 17.2 g of 3-cyano-naphthalene-1-carbonyl chloride (formula III, from reaction step A) were added and stirred for 3 hours. The organic solvents were eliminated and the remaining mixture was supplemented with ethyl acetate and methyl-tert.-butylether. The water phase was eliminated while the organic phase was washed four times with 50 ml of water and dried over sodium sulfate. The organic phase was concentrated to dryness, providing 31.8 g of a yellowish solid (3-cyano-naphthalene-1-carboxylic acid [2S-(3,4-dichloro-phenyl)-4-hydroxy-butyl]-methyl-amide; formula V) which was used without further purification.

C) 25 ml DMSO in 100 ml dichloromethane are added dropwise to 9.7 g oxalyl chloride in 100 ml dichloromethane unter nitrogen at - 70 °C unter stirring. The resulting was stired for another 15 minutes before 31.7 g of (3-cyano-naphthalene-1-carboxylic acid [2S-(3,4-dichloro-phenyl)-4-hydroxy-butyl]-methyl-amide (formula V, from reaction step B) in 200 ml dichloromethane and 6 ml DMSO were added. The mixture was stirred for another hour at - 70 °C. 52 ml of triethylamine in 50 ml dichloromethane were added dropwise. The solution was stirred at - 70 °C for 15 minutes and then allowed to warm up to room temperature. The solvent was removed and the residue was dissolved in 300 ml of toluene and 200 ml of ethyl acetate. The resulting solution was washed six times with 200 ml of a saturated solution of NaCl in water, dried over sodium sulfate and concentrated to dryness to deliver 31.0 g of [7-Cyano-naphthalene-2-carboxylic acid [(2*S*)-2-(3,4-dichloro-phenyl)-4-oxo-butyl]-methylamide (formula XVII).

D) 860 mg of (2*S*)-1-furan-2-yl-1-piperazin-1-yl-butan-2-ol dihydrochlorid (formula XII, from reaction step B) of Process 2), 860 mg of [7-Cyano-naphthalene-2-carboxylic acid [(2*S*)-2-(3,4-dichloro-phenyl)-4-oxo-butyl]-methyl-amide (formula XVII, from reaction step C)), 200 μl acetic acid and 0.1 ml water were put in suspension in 150 ml THF and stirred for four hours. 1.49 g sodium triacetoxyborohydride were added and the reaction mixture was stirred for 15 hours at room temperature. The solution was concentrated and 0.4 g KOH in 1 ml water as well as 10 ml methyl-tert.-butylether and 50 ml ethyl acetate were added. Water was eliminated and the organic pages was wash four times with 20 ml water, dried over sodium sulfate and concenteated to dryness to deliver 1.32 g of a foam. This foam was dissolved in 9 ml isopropyl alcohol, and heated to 60°C. A product crystallizes which was redissolved by adding 28 ml isopropyl alcohol (added in three portions) at 75°C. After cooling down to room temperature, crystals were obtained which were washed three times with 20 ml methyl-tert.-butylether to deliver 1.15 g 3-cyano-*N*-[(2*S*)-2-(3,4-dichlorophenyl)-4-{4-[(1*R*,2*S*)-1-(2-furyl)-2,3-dihydroxypropyl]piperazin-1-yl}butyl]-*N*-methyl-1-naphthamide monoisopropylate (formula I).
Melting point: 165 - 166 °C.

Example 5:

3-cyano-*N*-[(2*S*)-2-(3,4-dichlorophenyl)-4-{4-[(1*R*,2*S*)-1-(2-furyl)-2-oxo[1,3]dioxolan-4-yl]piperazin-1-yl}methyl]-*N*-methyl-1-naphthamide

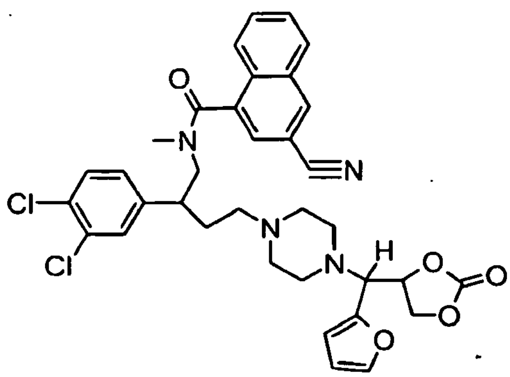

650 mg 3-cyano-*N*-[(2*S*)-2-(3,4-dichlorophenyl)-4-{4-[(1*R*,2*S*)-1-(2-furyl)-2,3-dihydroxypropyl]piperazin-1-yl} butyl]-*N*-methyl-1-naphthamide from any of the above processes were suspended in 50 ml dichloromethane at room temperature. 216 mg of N,N'-carbonydiimidazole in 60 ml dichloromethane were added over a period of 70 minutes. The solution was stirred for 5 hours at room temperature before being washed with 50 ml of a saturated solution of sodium hydrogencarbonat. The mixture was then washed with water until a pH of 6 was reached. Removal of the solvents

in vacuum lead to 634 mg of 3-cyano-*N*-[(2*S*)-2-(3,4-dichlorophenyl)-4-{4-[(1*R*,2*S*)-1-(2-furyl)-2-oxo[1,3]dioxolan-4-yl] piperazin-1-yl}methyl]-*N*-methyl-1-naphthamide.

Example 6:

3-cyano-*N*-[(2*S*)-2-(3,4-dichlorophenyl)-4-{4-[(1*R*,2*S*)-1-(2-furyl)-2-oxo[1,3]dioxolan-4-yl]piperazin-1-yl}methyl]-*N*-methyl-1-naphthamide monoacetate

350 mg 3-cyano-*N*-[(2*S*)-2-(3,4-dichlorophenyl)-4-{4-[(1*R*,2*S*)-1-(2-furyl)-2-oxo[1,3]dioxolan-4-yl]piperazin-1-yl}methyl]-*N*-methyl-1-naphthamide monoethanolate were dissolved under stirring in 50 ml ethyl acetate at room temperature under stirring. 120 μl of acetyl chloride and 250 μl of triethylamine were added successively. The reaction mixture was stirred further for 3 hours at room temperature and washed with 50 ml of a saturated solution of sodium carbonate in water and five times with 30 ml of water. After drying with sodium sulfate, the solution was concentrated to dryness to deliver 342 mg of a mixture which contains mainly monoacetic ester of the primary alcohol as seen in LC-MS and NMR. The mixture was purified by column chromatography on 10 g of $SiO_2$ with ethyl acetate/ethanol as mixture of eluents to deliver 136 mg of the monoacetate which was characterized in LC/MS and NMR.

Example 7:

3-cyano-*N*-[(2*S*)-2-(3,4-dichlorophenyl)-4-{4-[(1*R*,2*S*)-1-(2-furyl)-2-oxo[1,3]dioxolan-4-yl]piperazin-1-yl}methyl]-*N*-methyl-1-naphthamide monoacetate

230 mg 3-cyano-*N*-[(2*S*)-2-(3,4-dichlorophenyl)-4-{4-[(1*R*,2*S*)-1-(2-furyl)-2-oxo[1,3]dioxolan-4-yl]piperazin-1-yl}methyl]-*N*-methyl-1-naphthamide monoethanolate were dissolved in 10 ml of pyridine at room temperature. 150 μl of acetyl chloride in 10 ml of methylene chloride were added dropwise. The reaction mixture was stirred for 4 hours at room temperature and after addition of 10 ml of water, concentrated in vacuum. The residue was dissolved in 50 ml ethyl acetate and was washed 6 times with 20 ml water. LC-MS and NMR show the presence of diacetate as the main compound in the raw mixture. 150 mg of the mixture were fractionated by column chromatography on 10 g of $SiO_2$ to deliver 84 mg of the expected diester as confirmed by NMR and MS.

The compounds of formula I listed in table 8 below may be prepared according to the process described in the above examples or according to processes analogous thereto.

Table 8: Examples of compounds of formula I.

| Structure | $R^1$ | $R^2$ | $R^3$ | $R^4$ | $R^5$ | $R^7$ | $R^8$ | $R^9$ | $R^{10}$ | $R^{11}$ | k | l | m | n |
|---|---|---|---|---|---|---|---|---|---|---|---|---|---|---|
| 1 | $CH_3$ | Cl | Cl | 2-furanyl | H | H | - | - | - | H | 1 | 0 | 0 | 0 |
| 2 | $CH_3$ | Cl | Cl | 2-furanyl | H | H | H | H | - | H | 1 | 1 | 1 | 0 |
| 3 | $CH_3$ | Cl | Cl | 2-furanyl | H | H | H | H | - | H | 1 | 1 | 1 | 0 |
| 4 | $CH_3$ | Cl | Cl | 3-thiophen | H | H | H | - | H | H | 1 | 1 | 0 | 1 |
| 5 | $CH_3$ | Cl | Cl | 3-thiophen | H | H | H | - | H | H | 1 | 1 | 0 | 1 |
| 6 | $CH_3$ | Cl | Cl | 2-furanyl | $CH_2OH$ from $R^6$ | - | - | - | - | H | 0 | 0 | 0 | 0 |
| 7 | $CH_3$ | Cl | Cl | 2-furanyl | H | H | H | H | - | H | 1 | 1 | 0 | 0 |
| 8 | $CH_3$ | Cl | Cl | 3-thiophen | H | H | - | - | - | H | 1 | 0 | 0 | 0 |
| 9 | $CH_3$ | Cl | Cl | 2-thiophen | H | H | - | - | - | H | 1 | 0 | 0 | 0 |
| 10 | $CH_3$ | Cl | Cl | 3-thiophen | H | 5-ring carbonyl | | | | | - | - | - | - |
| 11 | $CH_3$ | Cl | Cl | 2-furanyl | H | 5-ring carbonyl | | | | | - | - | - | - |
| 12 | $CH_3$ | Cl | Cl | 3-furanyl | H | H | - | - | - | H | 1 | 0 | 0 | 0 |
| 13 | $CH_3$ | Cl | Cl | 2-furanyl | | 5-ring methylene | | | | | - | - | - | - |
| 14 | $CH_3$ | Cl | Cl | 2-furanyl | H | H | - | - | - | H | 1 | 0 | 0 | 0 |

(continued)

| Structure | $R^1$ | $R^2$ | $R^3$ | $R^4$ | $R^5$ | $R^7$ | $R^8$ | $R^9$ | $R^{10}$ | $R^{11}$ | k | l | m | n |
|---|---|---|---|---|---|---|---|---|---|---|---|---|---|---|
| 15 | $CH_3$ | Cl | Cl | 4-methyl-phenyl | H | H | - | - | - | H | 1 | 0 | 0 | 0 |
| 16 | $CH_3$ | Cl | Cl | 4-methoxy-phenyl | H | H | - | - | - | H | 1 | 0 | 0 | 0 |
| 17 | $CH_3$ | Cl | Cl | 2-benzo[b] furan | H | H | - | - | - | H | 1 | 0 | 0 | 0 |
| 18 | $CH_3$ | Cl | Cl | 5-chloro-2-thiophene | H | H | - | - | - | H | 1 | 0 | 0 | 0 |
| 19 | $CH_3$ | Cl | Cl | 2-methoxy-phenyl | H | H | - | - | - | H | 1 | 0 | 0 | 0 |
| 20 | $CH_3$ | Cl | Cl | 3-pyridine | H | H | - | - | - | H | 1 | 0 | 0 | 0 |
| 21 | $CH_3$ | Cl | Cl | 3,4-methylene-dioxyphenyl | H | H | - | - | - | H | 1 | 0 | 0 | 0 |
| 22 | $CH_3$ | Cl | Cl | 2-benzo[b] thio phene | H | H | - | - | - | H | 1 | 0 | 0 | 0 |
| 23 | $CH_3$ | Cl | Cl | phenyl | H | H | - | - | - | H | 1 | 0 | 0 | 0 |
| 24 | $CH_3$ | Cl | Cl | benzyl | H | H | - | - | - | H | 1 | 0 | 0 | 0 |
| 25 | $CH_3$ | Cl | Cl | 2-furanyl | H | - | - | - | - | H | 0 | 0 | 0 | 0 |
| 26 | $CH_3$ | Cl | Cl | benzyl | H | H | - | - | - | $COCH_3$ | 1 | 0 | 0 | 0 |
| 27 | $CH_3$ | Cl | Cl | 2-furanyl | H | $COCH_3$ | - | - | - | $COCH_3$ | 0 | 0 | 0 | 0 |
| 28 | $CH_3$ | Cl | Cl | 2-furanyl | H | - | - | - | - | H | 0 | 0 | 0 | 0 |

The compounds of Examples 15 to 22 listed' in table 8 above were also prepared using an automated preparation process. For this, per batch in each case 200 µl of a 0.25 N aqueous stock solution of the corresponding carbohydrate compound of formula IX was measured in a microreaction vessel and evaporated in a vacuum to largely remove the water. The residue was taken up in 200 µl ethanol. In each case 200 µl of a 0.25 mol/l ethanolic stock solution of racemic or enantiomerically pure (cf. in each case the corresponding particulars in table 7) N-[(2*S*)-2-(3,4-dichlorophenyl)-4-(1-piperazinyl)butyl]-N-methylbenzamide of formula VII and 200 µl of a 0.25 N ethanolic stock solution of the corresponding boronic acid (= dihydroxyborane compound) of formula VIII was added to this initial solution. The reaction mixture was first heated to 80°C for 2 h and then cooled to room temperature and 1 ml ethanol was added thereto. Then 100 mg basic Amberjet® ion exchange resin was added and the reaction vessel was shaken for 2 h. The ion exchanger was filtered off, was subsequently washed twice with 500 µl ethanol each time and the solvent was evaporated to dryness in a vacuum. Samples were taken from the residue without further purification in each case for high-performance liquid chromatography (= HPLC) and for automatic mass spectroscopy to determine the purity and to confirm the structure.

Example 8:

Capsules containing 3-cyano-*N*-[(2*S*)-2-(3,4-dichlorophenyl)-4-{4-[(1*R*,2*S*)-1-(2-furyl)-2,3-dihydroxypropyl]piperazin-1-yl}butyl]-*N*-methyl-1-naphthamide

Capsules with the following composition per capsule were produced:

| | |
|---|---|
| 3-cyano-*N*-[(2*S*)-2-(3,4-dichlorophenyl)-4-{4-[(1*R*,2*S*)-1-(2-furyl)-2,3-dihydroxypropyl]piperazin-1-yl} butyl]-*N*-methyl-1-naphthamide | 20 mg |
| Corn starch | 60 mg |
| Lactose | 300 mg |

(continued)

| | |
|---|---|
| Ethyl acetate | q.s. |

The active substance, the corn starch and the lactose were processed into a homogenous pasty mixture using EE. The paste was ground and the resulting granules were placed on a suitable tray and dried at 45°C in order to remove the solvent. The dried granules were passed through a crusher and mixed in a mixer with the further following auxiliaries:

| | |
|---|---|
| Talcum | 5 mg |
| Magnesium stearate | 5 mg |
| Corn starch | 9 mg |

and then poured into 400 mg capsules (= capsule size 0).

## Claims

1. Compounds of general formula I

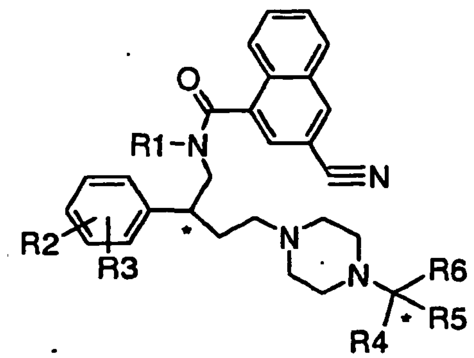

wherein

R1 is selected from the group consisting of: hydrogen and $C_1$ to $C_4$ alkyl,
R2 is halogen,
R3 is halogen,
R4 is selected from the group consisting of: 2-furanyl, 3-furanyl, 2-thiophene, 3-thiophene, phenyl, benzyl, 2-benzofuranyl, 3-benzofuranyl, 5-chloro-2-thiophene, 4-methylphenyl, 3,4-methylenedioxyphenyl, 2-methoxyphenyl, 4-methoxyphenyl, 2-pyridinyl, 3-pyridinyl, 1-benio[c]thiophene, 4-benzo[c]thiophene, 5-benzo[c]thiophene, 2-benzo[b]thiophene, 3-benzo[b]thiophene, 4-benzo[b]thiophene, 5-benzo[b]thiophene, 6-benzo[b]thiophene, 7-benzo[b]thiophene, 1-benzo[1,3]dioxole, 4-benzo[1,3]dioxole, and 5-benzo[1,3]dioxole, R5 is selected from the group consisting of: hydrogen and R6,
R6 represents a subgroup of the general formula

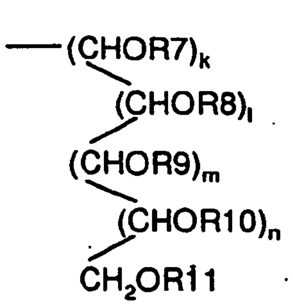

wherein

R7 is selected from the group consisting of: hydrogen or $C_1$ to $C_4$ alkanoyl, or together with another substituent, selected from the group consisting of R8, R9, R10 and R11, may form a 5- or 6-ring bridged by carbonyl, or by methylene optionally substituted by $C_1$ to $C_4$ alkyl or $C_4$ to $C_5$ alkylene,
R8 is selected from the group consisting of: hydrogen or $C_1$ to $C_4$ alkanoyl, or together with another substituent, selected from the group consisting of R7, R9, R10 and R11, may form a 5- or 6-ring bridged by carbonyl, or by methylene optionally substituted by $C_1$ to $C_4$ alkyl or $C_4$ to $C_5$alkylene,
R9 is selected from the group consisting of: hydrogen or $C_1$ to $C_4$ alkanoyl, or together with another substituent, selected from the group consisting of R7, R8, R10 and R11, may form a 5- or 6-ring bridged by carbonyl, or by methylene optionally substituted by $C_1$ to $C_4$ alkyl or $C_4$ to $C_5$ alkylene,
R10 is selected from the group consisting of: hydrogen or $C_1$ to $C_4$ alkanoyl, or together with another substituent, selected from the group consisting of R7, R8, R9 and R11, may form a 5- or 6-ring bridged by carbonyl, or by methylene optionally substituted by $C_1$ to $C_4$ alkyl or $C_4$ to $C_5$ alkylene,
R11 is selected from the group consisting of: hydrogen or $C_1$ to $C_4$ alkanoyl, or together with another substituent, selected from the group consisting of R7, R8, R9 and R10, may form a 5- or 6-ring bridged by carbonyl, or by methylene optionally substituted by $C_1$ to $C_4$ alkyl or $C_4$ to $C_5$ alkylene,
k is 0 or 1,
l is 0 or 1,
m is 0 or 1,
n is 0 or 1,

or physiologically compatible acid addition salts thereof.

**2.** Compounds according to Claim 1 wherein R1 represents methyl.

**3.** Compounds according to Claims 1 or 2 wherein R2 and R3 each represent chlorine.

**4.** Compounds according to any of the preceding claims wherein R4 is selected from the group consisting of: 2-furanyl, 3-furanyl, 2-thiophene, 3-thiophene, phenyl, benzyl, 2-benzofuranyl, 5-chloro-2-thiophene, 4-methylphenyl, 3,4-methylenedioxyphenyl, 2-methoxyphenyl and 4-methoxyphenyl.

**5.** Compounds according to any of the preceding claims wherein R4 is selected from the group consisting of: 2-furanyl, 3-furanyl, 2-thiophene, and 3-thiophene.

**6.** Compounds according to any of the preceding claims wherein R5 represents hydrogen.

**7.** Compounds according to any of the preceding claims wherein R7 and R11 each represent hydrogen; k represents 1; and l, m, n are each 0.

**8.** Compounds according to claim 1-6 wherein R7, R8 and R11 each represent hydrogen; k and l are each 1; and m and n are each 0.

**9.** Compounds according to claims 1-6 wherein R7, R8, R9 and R11 each represent hydrogen; k, l and m are each 1; and n is 0.

**10.** Compounds according claims 1-6 wherein R7 to R11 each represent hydrogen; k, l and m are each 1; and n is 0.

**11.** Compounds according to any of the preceding claims wherein the chiral centre *C is in the S configuration.

**12.** Compounds according to any of Claims 1 to 4 which are selected from the group consisting of:

3- cyano- *N*-[(2*S*)- 2-(3,4- dichlorophenyl)- 4- {4-[(1*R*, 2*S*)- 1-(2- furyl)- 2,3- dihydroxypropyl] piperazin- 1- yl} butyl]-*N*-methyl-1-naphthamide;
(2*S*, 3*R*)- 2-(acetyloxy)- 3- {4-[(3*S*)- 4-[3- cyano- 1- naphthoyl) (methyl) amino]- 3-(3,4- dichlorophenyl) butyl] piperazin-1-yl}-3-(2-furyl)propyl acetate;
3- cyano- *N*-[(2*S*)- 2-(3,4- dichlorophenyl)- 4- {4-[1-(2- furyl)- 2- hydroxyethyl] piperazin- 1- yl} butyt]-*N*-methyl- 1-naphthamide;
3-cyano-*N*-[(2*S*)-2-(3,4-dichlorophenyl)-4-{4-[(1*R*,2*S*,3*R*,4*R*)-1-(2-furyl)-2,3,4,5-tetrahydroxypentyl]piperazin-1-yl}butyl]-*N*-methyl-1-naphthamide;

3-cyano-*N*-[(2*S*)-2-(3,4-dichiorophenyl)-4-{4-[(1*R*,2*S*,3*S*,4*R*)-1-(2-furyl)-2,3,4,5-tetrahydroxypentyl]piperazin-1-yl}butyl]-*N*-methyl-1-naphthamide;
3-cyano-*N*-[(2*S*)-2-(3,4-dichlorophenyl)-4-{4-[(1*S*,2*S*,3*S*,4*R*)-2,3,4,5-tetrahydroxy-1-(3-thienyl)pentyl]piperazin-1-yl}butyl]-*N*-methyl-1-naphthamide;
3-cyano-*N*-[(2*S*)-2-(3,4-dichlorophenyl)-4-{4-[(1*S*,2*S*,3*R*,4*R*)-2,3,4,5-tetrahydroxy-1-(3-thienyl)pentyl]piperazin-1-yl}butyl]-*N*-Mmethyl-1-naphthamide;
3-cyano-*N*-[(2*S*)-2-(3,4-dichlorophenyl)-4-{4-[1-(2-furyl)-2-hydroxy-1-(hydroxymethyl)ethyl]piperazin-1-yl}butyl]-*N*-methyl-1-naphthamide;
3-cyano-*N*-[(2*S*)-2-(3,4-dichlorophenyl)-4-{4-[1-(2-furyl)-2-hydroxy-1-(hydroxymethyl)ethyl]piperazln-1-yl}butyl]-*N*-methyl-1-naphthamide;
3-cyano-*N*-[(2*S*)-2-(3,4-dichlorophenyl)-4-{4-[(1*S*, 2*S*)-2,3-dihydroxy-1-(3-thienyl)propyl]piperazin-1-yl}butyl]-*N*-methyl-1-naphthamide;
3-cyano-*N*-[(2*S*)-2-(3,4-dichlorophenyl)-4-14-[(1*R*, 2*S*)-2,3-dihydroxy-1-(2-thienyl)propyl]piperazin-1-yl}butyl]-*N*-methyl-1-naphthamide;
3-cyano-*N*-[(2*S*)-2-(3,4-dichlorophenyl)-4-{4-[(*S*)-[(4*S*)-2-oxo-1,3-dioxolan-4-yl](3-thienyl) methyl]piperazin-1-yl}butyl]-*N*-methyl-1-naphthamide;
3-cyano-*N*-[(2*S*)-2-(3,4-dichlorophenyl)-4-(4-{(*R*)-2-furyl[(4*S*)-2-oxo-1,3-dioxolan-4-yl]methyl}piperazin-1-yl)butyl]-*N*-methyl-1-naphthamide;
3-'cyano-*N*-(2*S*)-2-(3,4-dichlorophenyl)-4-{4-[(1*S*, 2*S*)-1-(3-furyl)-2,3-dihydroxypropyl]piperazln-1-yl}butyl]-*N*-methyl-1-naphthamlde:
3-cyano-*N*-[(2*S*)-2-(3,4-dichlorophenyl)-4-{4-[1-(2-furyl)-2,3-dihydroxypropyl]piperazin-1-yl}butyl]-*N*-methyl-1-naphthamide;
3-cyano-*N*-[(2*S*)-2-(3,4-dichlorophenyl)-4-{4-[(1*S*, 2*S*)-2,3-dihydroxyl-1-phenylpropyl]piperazin-1-yl}butyl]-*N*-methyl-1-naphthamide;
3-cyano-*N*-[(2)-2-(3,4-dichlorophenyl)-4-{4-[(1*S*,2*S*)-2,3-dihydroxy-1-(4-methoxyphenyl)propyl]piperazin-1-yl}butyl]-*N*-methyl-1-naphthamide;
N-[(2*S*)-4-{4-[(1*R*,2*S*)-1-(1-benzofuran-2-yl)-2,3-dihydroxypropyl]piperazin-1-yl}-2-(3,4-dichlorophenyl)butyl]-3-cyano-*N*-methy-1-naphthamide;
*N*-[(2*S*)-4-{4-[(1*R*,2*S*)-1-(5-chloro-2-thienyl)-2,3-dihydroxypropyl]piperazin-1-yl}-2-(3,4-dichlorophenyl)butyl]-3-cyano-*N*-methyl-1-naphthamide;
*N*-[(2*S*)-4-{4-[(1*S*,2*S*)-1-(1,3-benzodioxol-5-yl)-2,3-dihydroxypropyl]piperazin-1-yl}-2-(3,4-dichlorophenyl)butyl]-3-cyano-*N*-methyl-1-naphthamide; and
*N*-[(2*S*)-4-{4-[(1*R*,2*S*)-1-(1-benzothien-2-yl)-2,3-dihydroxypropy]piperazin-1-yl}-2-(3,4-dichlorophenyl)butyl]-3-cyano-*N*-methyl-1-naphthamide.

**13.** Pharmaceutical composition comprising a compound according to any of Claims 1 to 12 and conventional pharmaceutical auxiliaries and/or carriers.

**14.** Compounds according to any of Claims 1 to 12 for use in the treatment and/or prophylaxis of respiratory diseases, in particular asthma, bronchitis, cough, and rhinitis; skin diseases, in particular inflammatory skin reactions, allergic skin reactions, and psoriasis; arthropathy diseases, in particular arthritis, vasculitides and systemic lupus erythematosus; functional or inflammatory disorders in the gastrointestinal tract, in particular pseudomembranous colitis and diarrhoe; bleb diseases such as cystitis and interstitial cystitis: and migraine.

**15.** Compounds according to Claim 14 wherein the disorders are functional or inflammatory disorders in the lower intestinal tracts in mammals and humans which involve increased sensitivity to pain and/or impaired stool passage in the colon region.

**16.** Compounds according to Claim 14 wherein the disorder is IBS.

**17.** A process for the preparation of compounds of general formula I

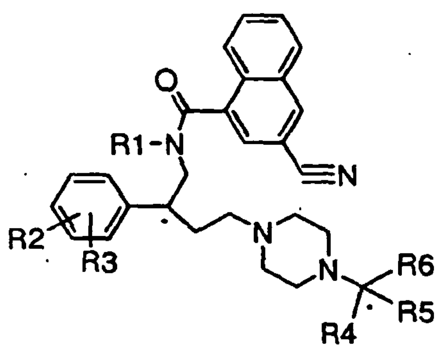

wherein

R1 is selected from the group consisting of: hydrogen and $C_1$ to $C_4$ alkyl,
R2 is halogen,
R3 is halogen,
R4 is selected from the group consisting of: 2-furanyl, 3-furanyl, 2-thiophene, 3-thiophene, phenyl, benzyl, 2-benzofuranyl, 3-benzofuranyl, 5-chloro-2-thiophene, 4-methylphenyl, 3,4-methylenedioxyphenyl, 2-methoxyphenyl, 4-methoxyphenyl, 2-pyridinyl, 3-pyridinyl, 1-benzo[c]thiophene, 4-benzo[c]thiophene, 5-benzo[c]thiophene, 2-benzo[b]thiophene, 3-benzo[b]thiophene, 4-benzo[b]thiophene, 5-benzo[b]thiophene, 6-kienzo[b]thiophene, 7-benzo[b]thiophene; 1-benzo[1,3]dioxole, 4-benzo[1,3]dioxole, and 5-benzo[1.3]dioxole,
R5 is selected from the group consisting of: hydrogen and R6,
R6 represents a subgroup of the general formula

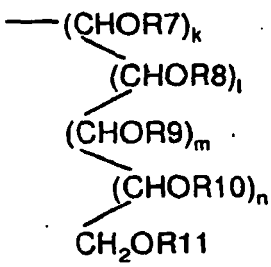

wherein

R7 is selected from the group consisting of: hydrogen or $C_1$ to $C_4$ alkanoyl, or together with another substituent, selected from the group consisting of R8, R9, R10 and R11, may form a 5- or 6- ring bridged by carbonyl, or by methylene optionally substituted by $C_1$ to $C_4$ alkyl or $C_4$ to $C_5$ alkylene,
R8 is selected from the group consisting of: hydrogen or $C_1$ to $C_4$ alkanoyl, or together with another substituent, selected from the group consisting of R7, R9, R10 and R11, may form a 5- or 6-ring bridged by carbonyl, or by methylene optionally substituted by $C_1$ to $C_4$ alkyl or $C_4$ to $C_5$alkylene,
R9 is selected from the group consisting of: hydrogen or $C_1$ to $C_4$ alkanoyl, or together with another substituent, selected from the group consisting of R7, R8, R10 and R11, may form a 5- or 6-ring bridged by carbonyl, or by methylene optionally substituted by $C_1$ to $C_4$ alkyl or $C_4$ to $C_5$ alkylene,
R10 Is selected from the group consisting of: hydrogen or $C_1$ to $C_4$ alkanoyl, or together with another substituent, selected from the group consisting of R7, R8, R9 and R11, may form a 5- or 6-ring bridged by carbonyl, or by methylene optionally substituted by $C_1$ to $C_4$ alkyl or $C_4$ to $C_5$ alkylene,
R11 is selected from the group consisting of: hydrogen or $C_1$ to $C_4$ alkanoyl, or together with another substituent, selected from the group consisting of R7, R8, R9 and R10, may form a 5- or 6-ring bridged by carbonyl, or by methylene optionally substituted by $C_1$ to $C_4$ alkyl or $C_4$ to $C_5$ alkylene,
k is 0 or 1,
l is 0 or 1,
m is 0 or 1,
n is 0 or 1,

or physiologically compatible acid addition salts thereof
**characterized in that**:

(a) a compound of the general formula VII

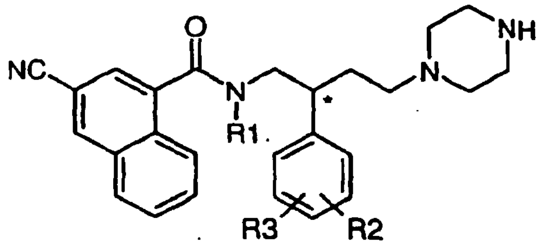

VII

is reacted with a compound of the general formula VIII,

R4 - $B(OH)_2$ VIII

and a compound of the general formula IX,

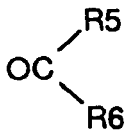

IX

to result in a compound of general formula I; or;
(b) a compound of general formula XV,

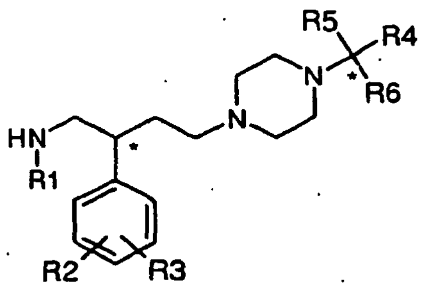

XV

is reacted with a compound of formula III

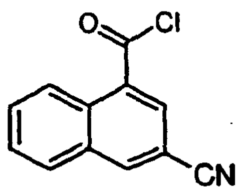

III

to result in a compound of general formula I,; or;
(c) a compound of general formula XVII

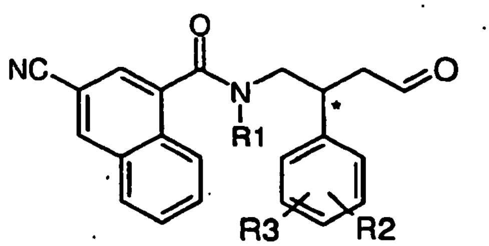

XVII

is reacted with a compound of general formula XII

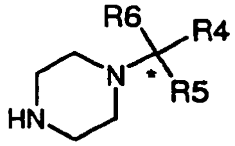

XII

to result in a compounds of general formula I;

which is optionally converted into its physiologically compatible acid addition salt.

**18.** Compounds of general formula VII

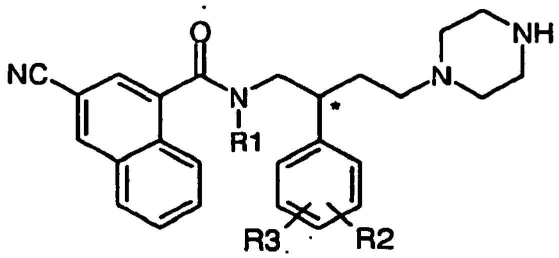

VII

wherein

R1 is selected from the group consisting of: hydrogen and $C_1$ to $C_4$ alkyl,
R2 is halogen and
R3 is halogen.

**19.** Compounds according to Claim 18 wherein R1 is methyl and wherein R2 and R3 each represent chlorine.

**20.** A compound according to any of claims 1 to 12 for use in a method of inhibiting a tachykinin mediate effect in a mammal.

**21.** Compound according to Claim 20 wherein the tachykinin is a neurokinin and the tachykinin receptor is an $NK_1$ or $NK_2$ receptor.

**22.** Compound according to Claim 20 wherein the tachykinin mediated effect is selected from the group consisting of hypotension, bronchoconstriction and colonic motility.

**Patentansprüche**

**1.** Verbindungen der allgemeinen Formel I

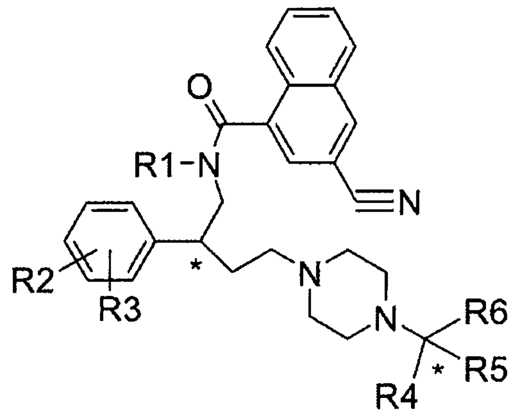

I

,

wobei

R1 aus der aus Wasserstoff und $C_1$- bis $C_4$-Alkyl bestehenden Gruppe ausgewählt ist,
R2 für Halogen steht,
R3 für Halogen steht,
R4 aus der aus 2-Furanyl, 3-Furanyl, 2-Thiophen, 3-Thiophen, Phenyl, Benzyl, 2-Benzofuranyl, 3-Benzofuranyl, 5-Chlor-2-thiophen, 4-Methylphenyl, 3,4-Methylendioxyphenyl, 2-Methoxyphenyl, 4-Methoxyphenyl, 2-Pyridinyl, 3-Pyridinyl, 1-Benzo[c]thiophen, 4-Benzo[c]thiophen, 5-Benzo [c] thiophen, 2-Benzo [b] thiophen, 3-Benzo [b] thiophen, 4-Benzo [b] thiophen, 5- Benzo [b] thiophen, 6-Benzo [b] thiophen, 7-Benzo [b] thiophen, 1-Benzo [1, 3] dioxol, 4-Benzo [1, 3] dioxol und 5-Benzo [1, 3] dioxol bestehenden Gruppe ausgewählt ist,
R5 aus der aus Wasserstoff und R6 bestehenden Gruppe ausgewählt ist,
R6 eine Untergruppe der allgemeinen Formel

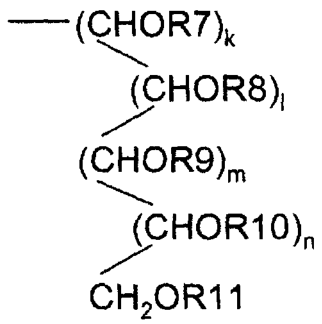

darstellt, wobei

R7 aus der aus Wasserstoff und $C_1$- bis $C_4$-Alkanoyl bestehenden Gruppe ausgewählt ist oder zusammen mit einem anderen Substituenten ausgewählt aus der aus R8, R9, R10 und R11 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann,
R8 aus der aus Wasserstoff und $C_1$- bis $C_4$-Alkanoyl bestehenden Gruppe ausgewählt ist oder zusammen mit einem anderen Substituenten ausgewählt aus der aus R7, R9, R10 und R11 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann,
R9 aus der aus Wasserstoff und $C_1$- bis $C_4$-Alkanoyl bestehenden Gruppe ausgewählt ist oder zusammen mit einem anderen Substituenten ausgewählt aus der aus R7, R8, R10 und R11 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann,
R10 aus der aus Wasserstoff und $C_1$- bis $C_4$-Alkanoyl bestehenden Gruppe ausgewählt ist oder zusammen mit einem anderen Substituenten ausgewählt aus der aus R7, R8, R9 und R11 bestehenden Gruppe einen durch Carbonyl oder durch gegebenenfalls durch $C_1$-bis $C_4$-Alkyl oder $C_4$- bis $C_5$-Alkylen substituiertes Methylen

verbrückten 5- oder 6-gliedrigen Ring bilden kann,
R11 aus der aus Wasserstoff und $C_1$- bis $C_4$-Alkanoyl bestehenden Gruppe ausgewählt ist oder zusammen mit einem anderen Substituenten ausgewählt aus der aus R7, R8, R9 und R10 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann.
k für 0 oder 1 steht,
l für 0 oder 1 steht,
m für 0 oder 1 steht,
n für 0 oder 1 steht,

und deren physiologisch unbedenkliche Säureadditionssalze.

2. Verbindungen nach Anspruch 1, wobei R1 für Methyl steht.

3. Verbindungen nach Anspruch 1 oder 2, wobei R2 und R3 jeweils für Chlor stehen.

4. Verbindungen nach einem der vorhergehenden Ansprüche, wobei R4 aus der aus 2-Furanyl, 3-Furanyl, 2-Thiophen, 3-Thiophen, Phenyl, Benzyl, 2-Benzofuranyl, 5-Chlor-2-thiophen, 4-Methylphenyl, 3,4-Methylendioxyphenyl, 2-Methoxyphenyl und 4-Methoxyphenyl bestehenden Gruppe ausgewählt ist.

5. Verbindungen nach einem der vorhergehenden Ansprüche, wobei R4 aus der aus 2-Furanyl, 3-Furanyl, 2-Thiophen und 3-Thiophen bestehenden Gruppe ausgewählt ist.

6. Verbindungen nach einem der vorhergehenden Ansprüche, wobei R5 für Wasserstoff steht.

7. Verbindungen nach einem der vorhergehenden Ansprüche, wobei R7 und R11 jeweils für Wasserstoff stehen; k für 1 steht; und 1, m, n jeweils für 0 stehen.

8. Verbindungen nach einem der Ansprüche 1 bis 6, wobei R7, R8 und R11 jeweils für Wasserstoff stehen; k und 1 jeweils für 1 stehen; und m und n jeweils für 0 stehen.

9. Verbindungen nach einem der Ansprüche 1 bis 6, wobei R7, R8, R9 und R11 jeweils für Wasserstoff stehen; k, 1 und m jeweils für 1 stehen; und n für 0 steht.

10. Verbindungen nach einem der Ansprüche 1 bis 6, wobei R7 bis R11 jeweils für Wasserstoff stehen; k, 1 und m jeweils für 1 stehen; und n für 0 steht.

11. Verbindungen nach einem der vorhergehenden Ansprüche, wobei das Chiralitätszentrum *C in der S-Konfiguration vorliegt.

12. Verbindungen nach einem der Ansprüche 1 bis 4, ausgewählt aus der aus

3-Cyano-*N*-[(2*S*)-2-(3,4-dichlorphenyl)-4-[4-[(1*R*,2*S*)-1-(2-furyl)-2,3-dihydroxypropyl]piperazin-1-yl]butyl-*N*-methyl-1-naphthamid;
(2*S*,3*R*)-2-(Acetyloxy)-3-{4-[(3*S*)-4-(3-cyano-1-naphthoyl)(methyl)amino]-3-(3,4-dichlorphenyl)butyl]piperazin-1-yl}-3-(2-furyl)propylacetat;
3-Cyano-*N*-[(2*S*)-2-(3,4-dichlorphenyl)-4-{4-[1-(2-furyl)-2-hydroxyethyl]piperazin-1-yl}butyl]-*N*-methyl-1-naphthamid;
3-Cyano-*N*-[(2*S*)-2-(3,4-dichlorphenyl)-4-{4-[(1*R*,2*S*,3*R*,4*R*)-1-(2-furyl)-2,3,4,5-tetrahydroxypentyl]piperazin-1-yl}butyl]-*N*-methyl-1-naphthamid;
3-Cyano-*N*-[(2*S*)-2-(3,4-dichlorphenyl)-4-{4-[(1*R*,2*S*,3*R*,4*R*)-1-(2-furyl)-2,3,4,5-tetrahydroxypentyl]piperazin-1-yl}butyl]-*N*-methyl-1-naphthamid;
3-Cyano-*N*-[(2*S*)-2-(3,4-dichlorphenyl)-4-{4-[(1*S*,2*S*,3*S*,4*R*)-2,3,4,5-tetrahydroxy-1-(3-thienyl)pentyl]piperazin-1-yl}butyl]-*N*-methyl-1-naphthamid;
3-Cyano-*N*-[(2*S*)-2-(3,4-dichlorphenyl)-4-{4-[(1*S*,2*S*,3*R*,4*R*)-2,3,4,5-tetrahydroxy-1-(3-thienyl)pentyl]piperazin-1-yl}butyl]-*N*-methyl-1-naphthamid;
3-Cyano-*N*-[(2*S*)-2-(3,4-dichlorphenyl)-4-{4-[1-(2-furyl)-2-hydroxy-1-(hydroxymethyl)ethyl]piperazin-1-yl}butyl]-*N*-methyl-1-naphthamid;

3-Cyano-*N*-[(2*S*)-2-(3,4-dichlorphenyl)-4-{4-[1-(2-furyl)-2-hydroxy-1-(hydroxymethyl)ethyl]piperazin-1-yl}butyl]-*N*-methyl-1-naphthamid;
3-Cyano-*N*-[(2*S*)-2-(3,4-dichlorphenyl)-4-{4-[(1*S*,2*S*)-2,3-dihydroxy-1-(3-thienyl)propyl]piperazin-1-yl}butyl]-*N*-methyl-1-naphthamid;
3-Cyano-*N*-[(2*S*)-2-(3,4-dichlorphenyl)-4-{4-[(1R,2S)-2,3-dihydroxy-1-(2-thienyl)propyl]piperazin-1-yl}butyl]-*N*-methyl-1-naphthamid;
3-Cyano-*N*-[(2*S*)-2-(3,4-dichlorphenyl)-4-{4-[(*S*)-[(4*S*)-2-oxo-1,3-dioxolan-4-yl](3-thienyl)methyl}piperazin-1-yl)butyl]-*N*-methyl-1-naphthamid;
3-Cyano-*N*-[(25)-2-(3,4-dichlorphenyl)-4-(4-{(*R*)-2-furyl[(4*S*)-2-oxo-1,3-dioxolan-4-yl]methyl}piperazin-1-yl)butyl]-*N*-methyl-1-naphthamid;
3-Cyano-*N*-[(2*S*)-2-(3,4-dichlorphenyl)-4-{4-[(1*S*,2*S*)-1-(3-furyl)-2,3-dihydroxypropyl]piperazin-1-yl}butyl]-*N*-methyl-1-naphthamid;
3-Cyano-*N*-[(2*S*)-2-(3,4-dichlorphenyl)-4-{4-[1-(2-furyl)-2,3-dihydroxypropyl]piperazin-1-yl}butyl]-*N*-methyl-1-naphthamid;
3-Cyano-*N*-[(2*S*)-2-(3,4-dichlorphenyl)-4-{4-[(1*S*,2*S*)-2,3-dihydroxy-1-phenylpropyl]piperazin-1-yl}butyl]-*N*-methyl-1-naphthamid;
3-Cyano-*N*-[(2)-2-(3,4-dichlorphenyl)-4-{4-[(1*S*,2*S*)-2,3-dihydroxy-1-(4-methoxyphenyl)propyl]piperazin-1-yl}butyl]-N-methyl-1-naphthamid;
*N*-[(2*S*)-4-{4-[(1*R*,2*S*)-1-(1-benzofuran-2-yl)-2,3-dihydroxypropyl]piperazin-1-yl}-2-(3,4-dichlorphenyl)butyl]-3-cyano-*N*-methyl-1-naphthamid;
*N*-[(2*S*)-4-{4-[(1*R*,2*S*)-1-(5-chlor-2-thienyl)-2,3-dihydroxypropyl]piperazin-1-yl}-2-(3,4-dichlorphenyl)butyl]-3-cyano-*N*-methyl-1-naphthamid;
*N*-[(2*S*)-4-{4-[(1*S*,2*S*)-1-(1,3-benzodioxol-5-yl)-2,3-dihydroxypropyl]piperazin-1-yl}-2-(3,4-dichlorphenyl)butyl]-3-cyano-*N*-methyl-1-naphthamid;
*N*-[(2*S*)-4-{4-[(1*R*,2*S*)-1-(1-benzothien-2-yl)-2,3-dihydroxypropyl]piperazin-1-yl}-2-(3,4-dichlorphenyl)butyl]-3-cyano-*N*-methyl-1-naphthamid

bestehenden Gruppe.

**13.** Pharmazeutische Zusammensetzung, enthaltend eine Verbindung nach einem der Ansprüche 1 bis 12 und herkömmliche pharmazeutische Hilfsstoffe und/oder Träger.

**14.** Verbindungen nach einem der Ansprüche 1 bis 12 zur Verwendung bei der Behandlung und/oder Prophylaxe von Atemwegserkrankungen, insbesondere Asthma, Bronchitis, Husten und Rhinitis; Hautkrankheiten, insbesondere entzündlichen Hautreaktionen, allergischen Hautreaktionen und Psoriasis; Arthropathiekrankheiten, insbesondere Arthritis, vaskulitidem und systemischem Lupus erythematodes; funktionellen oder entzündlichen Erkrankungen des Magen-Darm-Trakts, insbesondere pseudomembranöser Kolitis und Diarrhoe; Blasenerkrankungen wie Cystitis und interstitieller Cystitis; und Migräne.

**15.** Verbindungen nach Anspruch 14, wobei es sich bei den Erkrankungen um funktionelle oder entzündliche Erkrankungen des unteren Darmtrakts in Säugetieren und Menschen handelt, bei denen es zu einer erhöhten Schmerzempfindlichkeit und/oder einer gestörten Stuhlpassage im Bereich des Dickdarms kommt.

**16.** Verbindungen nach Anspruch 14, wobei es sich bei der Erkrankung um Reizkolon handelt.

**17.** Verfahren zur Herstellung von Verbindungen der allgemeinen Formel I

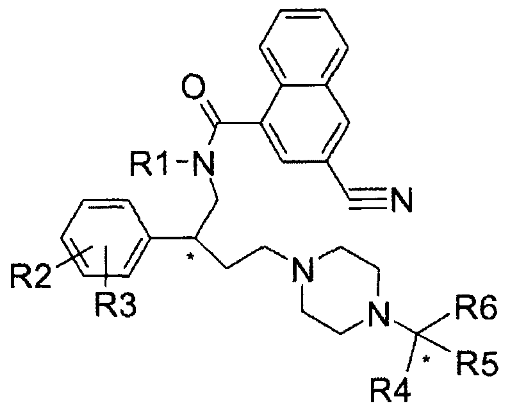

I

,

wobei

R1 aus der aus Wasserstoff und $C_1$- bis $C_4$-Alkyl bestehenden Gruppe ausgewählt ist,
R2 für Halogen steht,
R3 für Halogen steht,
R4 aus der aus 2-Furanyl, 3-Furanyl, 2-Thiophen, 3-Thiophen, Phenyl, Benzyl, 2-Benzofuranyl, 3-Benzofuranyl, 5-Chlor-2-thiophen, 4-Methylphenyl, 3,4-Methylendioxyphenyl, 2-Methoxyphenyl, 4-Methoxyphenyl, 2-Pyridinyl, 3-Pyridinyl, 1-Benzo [c] thiophen, 4-Benzo [c] thiophen, 5-Benzo [c] thiophen, 2-Benzo [b] thiophen, 3-Benzo [b] thiophen, 4-Benzo [b] thiophen, 5-Benzo [b] thiophen, 6-Benzo [b] thiophen, 7-Benzo [b] thiophen, 1-Benzo [1,3] dioxol, 4- Benzo [1,3] dioxol und 5-Benzo [1,3] dioxol bestehenden Gruppe ausgewählt ist,
R5 aus der aus Wasserstoff und R6 bestehenden Gruppe ausgewählt ist,
R6 eine Untergruppe der allgemeinen Formel

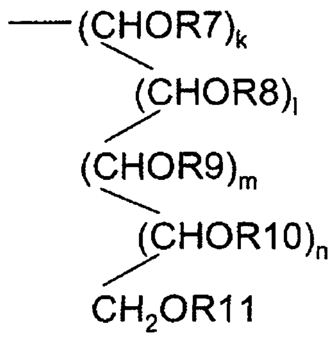

darstellt, wobei

R7 aus der aus Wasserstoff und $C_1$- bis $C_4$-Alkanoyl bestehenden Gruppe ausgewählt ist oder zusammen mit einem anderen Substituenten ausgewählt aus der aus R8, R9, R10 und R11 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann,
R8 aus der aus Wasserstoff und $C_1$- bis $C_4$-Alkanoyl bestehenden Gruppe ausgewählt ist oder zusammen mit einem anderen Substituenten ausgewählt aus der aus R7, R9, R10 und R11 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann,
R9 aus der aus Wasserstoff und $C_1$- bis $C_4$-Alkanoyl bestehenden Gruppe ausgewählt ist oder zusammen mit einem anderen Substituenten ausgewählt aus der aus R7, R8, R10 und R11 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann,
R10 aus der aus Wasserstoff und $C_1$- bis $C_4$-Alkanoyl bestehenden Gruppe ausgewählt ist oder zusammen mit einem anderen Substituenten ausgewählt aus der aus R7, R8, R9 und R11 bestehenden Gruppe einen durch Carbonyl oder durch gegebenenfalls durch $C_1$-bis $C_4$-Alkyl oder $C_4$- bis $C_5$-Alkylen substituiertes Methylen

verbrückten 5- oder 6-gliedrigen Ring bilden kann,
R11 aus der aus Wasserstoff und $C_1$- bis $C_4$-Alkanoyl bestehenden Gruppe ausgewählt ist oder zusammen mit einem anderen Substituenten ausgewählt aus der aus R7, R8, R9 und R10 bestehenden Gruppe einen durch Carbonyl oder durch gegebenenfalls durch $C_1$-bis $C_4$-Alkyl oder $C_4$- bis $C_5$-Alkylen substituiertes Methylen verbrückten 5- oder 6-gliedrigen Ring bilden kann.
k für 0 oder 1 steht,
1 für 0 oder 1 steht,
m für 0 oder 1 steht,
n für 0 oder 1 steht,

und deren physiologisch unbedenkliche Säureadditionssalze
**dadurch gekennzeichnet, dass** man:

(a) eine Verbindung der allgemeinen Formel VII

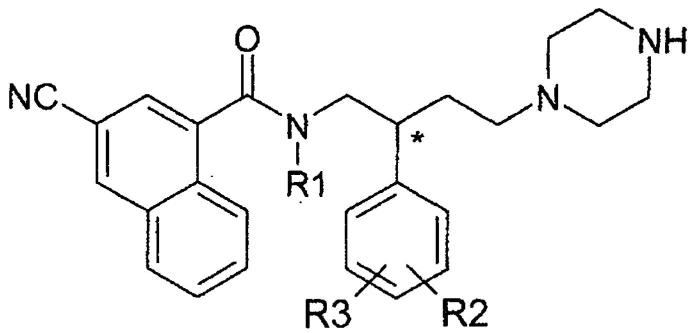

VII

mit einer Verbindung der allgemeinen Formel VIII

$R4 - B(OH)_2$ VIII

und einer Verbindung der allgemeinen Formel IX

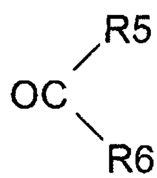

IX

zu einer Verbindung der allgemeinen Formel I umsetzt; oder
(b) eine Verbindung der allgemeinen Formel XV

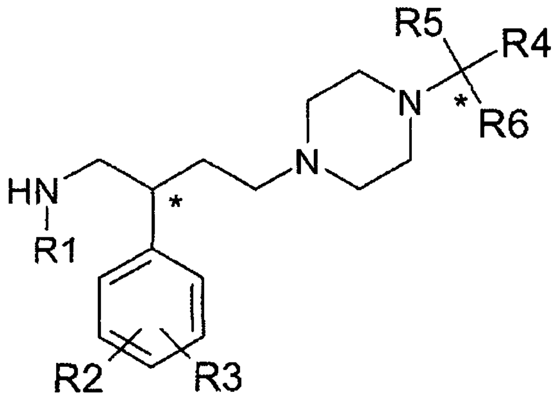

XV

mit einer Verbindung der Formel III

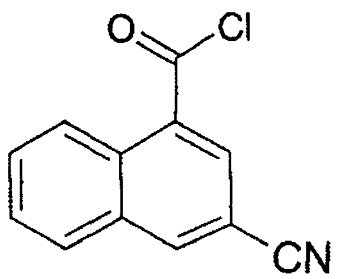

III

zu einer Verbindung der allgemeinen Formel I umsetzt; oder

(c) eine Verbindung der allgemeinen Formel XVII

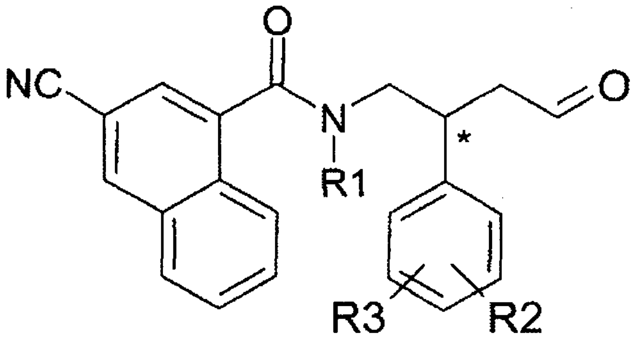

XVII

mit einer Verbindung der allgemeinen Formel XII

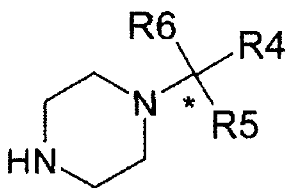

XII

zu einer Verbindung der allgemeinen Formel I umsetzt;

welche gegebenenfalls in ihr physiologisch unbedenkliches Säureadditionssalz umgewandelt wird.

**18.** Verbindungen der allgemeinen Formel VII

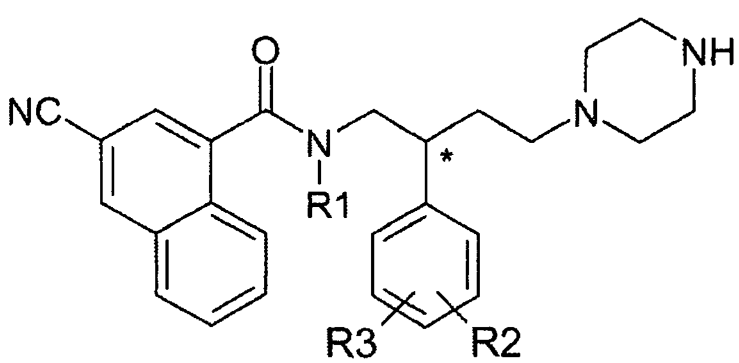

VII

,

wobei
R1 aus der aus Wasserstoff und $C_1$- bis $C_4$-Alkyl bestehenden Gruppe ausgewählt ist,
R2 für Halogen steht und
R3 für Halogen steht.

**19.** Verbindung nach Anspruch 18, wobei R1 für Methyl steht und wobei R2 und R3 jeweils für Chlor stehen.

**20.** Eine Verbindung nach einem der Ansprüche 1 bis 12 zur Verwendung bei einem Verfahren zur Inhibierung einer durch Tachykinin vermittelten Wirkung in einem Säugetier.

**21.** Verbindung nach Anspruch 20, wobei es sich bei dem Tachykinin um ein Neurokinin handelt und es sich bei dem Tachykininrezeptor um einen $NK_1$- oder $NK_2$-Rezeptor handelt.

**22.** Verbindung nach Anspruch 20, wobei die tachykininvermittelte Wirkung aus der aus Hypotonie, Bronchokonstriktion und Kolonmotilität bestehenden Gruppe ausgewählt ist.

**Revendications**

**1.** Composés de formule générale 1

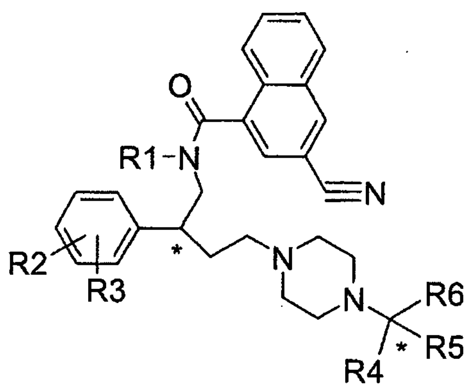

I

dans laquelle

R1 est choisi dans le groupe constitué par hydrogène et alkyle en $C_1$ à $C_4$,
R2 est halogène,
R3 est halogène,
R4 est choisi dans le groupe constitué par : 2-furanyle, 3-furanyle, 2-thiophène, 3-thiophène, phényle, benzyle, 2-benzofuranyle, 3-benzofuranyle, 5-chloro-2-thiophène, 4-méthylphényle, 3,4-méthylènedioxyphényle, 2-méthoxyphényle, 4-méthoxyphényle, 2-pyridinyle, 3-pyridinyle, 1-benzo[c]thiophène, 4-benzo[c]thiophène, 5-benzo[c]thiophène, 2-benzo[b]thiophène, 3-benzo[b]thiophène, 4-benzo[b]thiophène, 5-benzo[b]thiophène, 6-benzo [b] thiophène, 7-benzo [b] thiophène, 1-benzo[1,3]dioxole, 4-benzo[1,3]dioxole, et 5-benzo [1,3] dioxole,
R5 est choisi dans le groupe constitué par hydrogène et R6,
R6 représente un sous-groupe de formule générale

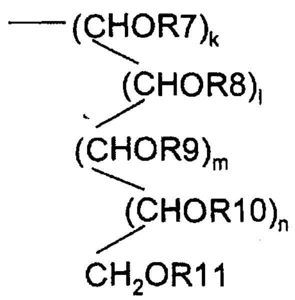

dans laquelle

R7 est choisi dans le groupe constitué par hydrogène ou alcanoyle en $C_1$ à $C_4$ ou conjointement avec un autre substituant, choisi parmi le groupe constitué par R8, R9, R10 et R11, peut former un cycle à 5 ou 6 chaînons ponté par carbonyle ou par méthylène éventuellement substitué par alkyle en $C_1$ à $C_4$ ou alkylène en $C_4$ à $C_5$,
R8 est choisi dans le groupe constitué par hydrogène ou alcanoyle en $C_1$ à $C_4$, ou conjointement avec un autre substituant, choisi parmi le groupe constitué par R7 R9, R10 et R11, peut former un cycle à 5 ou 6 chaînons ponté par carbonyle ou par méthylène éventuellement substitué par alkyle en $C_1$ à $C_4$ ou alkylène en $C_4$ à $C_5$,
R9 est choisi dans le groupe constitué par hydrogène ou alcanoyle en $C_1$ à $C_4$, ou conjointement avec un autre substituant, choisi parmi le groupe constitué par R7 R8, R10 et R11, peut former un cycle à 5 ou 6 chaînons ponté par carbonyle ou par méthylène éventuellement substitué par alkyle en $C_1$ à $C_4$ ou alkylène en $C_4$ à $C_5$,
R10 est choisi dans le groupe constitué par hydrogène ou alcanoyle en $C_1$ à $C_4$, ou conjointement avec un autre substituant, choisi parmi le groupe constitué par R7 R8, R9 et R11, peut former un cycle à 5 ou 6 chaînons ponté par carbonyle ou par méthylène éventuellement substitué par alkyle en $C_1$ à $C_4$ ou alkylène en $C_4$ à $C_5$,
R11 est choisi dans le groupe constitué par hydrogène ou alcanoyle en $C_1$ à $C_4$, ou conjointement avec un autre substituant, choisi parmi le groupe constitué par R7 R8, R9 et R10, peut former un cycle à 5 ou 6 chaînons ponté par carbonyle ou par méthylène éventuellement substitué par alkyle en $C_1$ à $C_4$ ou alkylène en $C_4$ à $C_5$,
k est 0 ou 1,
1 est 0 ou 1,
m est 0 ou 1,
n est 0 ou 1,

ou les sels d'addition d'acide physiologiquement compatibles de ceux-ci.

**2.** Composés selon la revendication 1, **caractérisés en ce que** R1 représente méthyle.

**3.** Composés selon la revendication 1 ou 2, **caractérisés en ce que** R2 et R3 représentent chacun le chlore.

**4.** Composés selon l'une quelconque des revendications précédentes, **caractérisés en ce que** R4 est choisi dans le groupe constitué par : 2-furanyle, 3-furanyle, 2-thiophène, 3-thiophène, phényle, benzyle, 2-benzofuranyle, 5-chloro-2-thiophène, 4-méthylphényle, 3,4-méthylènedioxyphényle, 2-méthoxyphényle et 4-méthoxyphényle.

**5.** Composés selon l'une quelconque des revendications précédentes, **caractérisés en ce que** R4 est choisi dans le groupe constitué par : 2-furanyle, 3-furanyle, 2-thiophène, et 3-thiophène.

**6.** Composés selon l'une quelconque des revendications précédentes, **caractérisés en ce que** R5 représente hydrogène.

**7.** Composés selon l'une quelconque des revendications précédentes, **caractérisés en ce que** R7 et R11 représentent chacun hydrogène ; k représente 1 ; et l, m, n sont chacun 0.

**8.** Composés selon les revendications 1 à 6, **caractérisés en ce que** R7, R8 et R11 représentent chacun hydrogène ; k et 1 sont chacun 1 et m et n sont chacun 0.

**9.** Composés selon les revendications 1 à 6, **caractérisés en ce que** R7, R8, R9 et R11 représentent chacun hydrogène ; k, l et m sont chacun 1 ; et n est 0.

**10.** Composés selon les revendications 1 à 6, **caractérisés en ce que** R7 à R11 représentent chacun hydrogène ; k, l et m sont chacun 1 ; et n est 0.

**11.** Composés selon l'une quelconque des revendications précédentes, **caractérisés en ce que** le centre chiral *C est dans la configuration S.

**12.** Composés selon l'une quelconque des revendications 1 à 4, **caractérisés en ce qu'**ils sont choisis dans le groupe constitué par :

3-cyano-*N*-[(2*S*)-2-(3,4-dichlorophényl)-4-{4-[(1*R*, 2*S*)-1-(2-furyl)-2,3-dihydroxypropyl]pipérazin-1-yl}butyl]-*N*-méthyl-1-naphtamide ;
acétate de (2*S*, 3*R*)-2-(acétyloxy)-3-{4-[(3*S*)-4-(3-cyano-1-naphtoyl)(méthyl)amino]-3-(3,4-dichlorophényl)butyl]pipérazin-1-yl}-3-(2-furyl)propyle ;
3- cyano- *N*-[(2*S*)- 2-(3,4- dichlorophényl)- 4- {4-[1-(2- furyl)- 2- hydroxyéthyl] pipérazin- 1- yl} butyl]-*N*- méthyl- 1-naphtamide ;
3-cyano-*N*-[(2*S*)-2-(3,4-dichlorophényl)-4-{4-[(1*R*,2*S*,3*R*,4*R*)-1-(2-furyl)-2,3,4,5-tétrahydroxypentyl]pipérazin-1-yl}butyl]-*N*-méthyl-1-naphtamide ;
3-cyano-*N*-[(2*S*)-2-(3,4-dichlorophényl)-4-{4-[(1*R*, 2*S*, 3*S*, 4*R*)-1-(2-furyl)-2, 3, 4, 5-tétrahydroxypentyl]pipérazin-1-yl}butyl]-*N*-méthyl-1-naphtamide ;
3-cyano-*N*-[(2*S*)-2-(3,4-dichlorophényl)-4-{4-[(1*S*,2*S*,3*S*,4*R*)-2,3,4,5-tétrahydroxy-1-(3-thiényl)pentyl]pipérazin-1-yl}butyl]-*N*-méthyl-1-naphtamide ;
3-cyano-*N*-[(2*S*)-2-(3,4-dichlorophényl)-4-{4-[(1*S*,2*S*,3*R*,4*R*)-2,3,4,5-tétrahydroxy-1-(3-thiényl)pentyl]pipérazin-1-yl}butyl]-*N*-méthyl-1-naphtamide ;
3-cyano-*N*-[(2*S*)-2-(3,4-dichlorophényl)-4-{4-[1-(2-furyl)-2-hydroxy-1-(hydroxyméthyl)éthyl]pipérazin-1-yl}butyl]-*N*-méthyl-1-naphtamide ;
3-cyano-*N*-[(2*S*)-2-(3,4-dichlorophényl)-4-{4-[1-(2-furyl)-2-hydroxy-1-(hydroxyméthyl)éthyl]pipérazin-1-yl}butyl]-*N*-méthyl-1-naphtamide ;
3- cyano- *N*-[(2*S*)- 2-(3,4- dichlorophényl)- 4- {4-[(1*S*, 2*S*)- 2,3- dihydroxy- 1-(3- thiényl) propyl] pipérazin- 1- yl} butyl]-*N*-méthyl-1-naphtamide ;
3- cyano- *N*-[(2*S*)- 2-(3,4- dichlorophényl)- 4- {4-[(1*R*, 2*S*)- 2,3- dihydroxy- 1-(2- thiényl) propyl] pipérazin- 1- yl} butyl]-*N*-méthyl-1-naphtamide ;
3-cyano-*N*-[(2*S*)-2-(3,4-dichlorophényl)-4-{4-[(*S*)-[(4*S*)-2-oxo-1,3-dioxolan-4-yl](3-thiényl)méthyl]pipérazin-1-yl}butyl]-*N*-méthyl-1-naphtamide ;
3-cyano-*N*-[(2*S*)-2-(3,4-dichlorophényl)-4-(4-{(*R*)-2-furyl[(4*S*)-2-oxo-1,3-dioxolan-4-yl]méthyl}pipérazin-1-yl)butyl]-*N*-méthyl-1-naphtamide ;
3- cyano- *N*-[(2*S*)- 2-(3,4- dichlorophényl)- 4- {4-[(1*S*, 2*S*)- 1-(3- furyl)- 2,3- dihydroxypropyl] pipérazin- 1- yl} butyl]-*N*-méthyl-1-naphtamide ;
3-cyano-*N*-[(2*S*)-2-(3,4-dichlorophényl)-4-{4-[1-(2-furyl)-2,3-dihydroxypropyl]pipérazin-1-yl}butyl]-*N*-méthyl-1-naphtamide ;
3- cyano- *N*-[(2*S*)- 2-(3,4- dichlorophényl)- 4- {4-[(1*S*, 2*S*)- 2,3- dihydroxy- 1- phénylpropyl] pipérazin- 1- yl} butyl]-*N*-méthyl-1-naphtamide ;
3-cyano-*N*-[(2)-2-(3,4-dichlorophényl)-4-{4-[(1*S*,2*S*)-2,3-dihydroxy-1-(4-méthoxyphényl)propyl]pipérazin-1-yl}butyl]-*N*-méthyl-1-naphtamide ;
*N*-[(2*S*)-4-{4-[(1*R*,2*S*)-1-(1-benzofuran-2-yl)-2,3-dihydroxypropyl]pipérazin-1-yl}2-(3,4-dichlorophényl)butyl]-3-cyano-*N*-méthyl-1-naphtamide ;
*N*-[(2*S*)-4-{4-[(1*R*,2*S*)-1-(5-chloro-2-thiényl)-2,3-dihydroxypropyl]pipérazin-1-yl}2-(3,4-dichlorophényl)butyl]-3-cyano-*N*-méthyl-1-naphtamide ;
N-[(2*S*)-4-{4-[(1*S*,2*S*)-1-(1,3-benzodioxol-5-yl)-2,3-dihydroxypropyl]pipérazin-1-yl}-2-(3,4-dichlorophényl)butyl]-3-cyano-*N*-méthyl-1-naphtamide ; et
*N*-[(2*S*)-4-{4-[(1*R*,2*S*)-1-(1-benzothién-2-yl)-2,3-dihydroxypropyl]pipérazin-1-yl}-2-(3,4-dichlorophényl)butyl]-3-cyano-N-méthyl-1-naphtamide.

**13.** Composition pharmaceutique comprenant un composé selon l'une quelconque des revendications 1 à 12 et des auxiliaires et/ou des supports pharmaceutiques classiques.

**14.** Composés selon l'une quelconque des revendications 1 à 12, destinés à une utilisation dans le traitement et/ou la prophylaxie des maladies respiratoires, en particulier de l'asthme, de la bronchite, de la toux et de la rhinite ; des maladies cutanées en particulier des réactions cutanées inflammatoires, des réactions cutanées allergiques et du

psoriasis ; des maladies arthropatiques, en particulier de l'arthrite, des vascularites et du lupus érythémateux disséminé ; des troubles fonctionnels ou inflammatoires dans le tractus gastro-intestinal, en particulier de la colite pseudo-membraneuse et de la diarrhée ; des maladies bulleuses telles que la cystite et la cystite interstitielle ; et de la migraine.

**15.** Composés selon la revendication 14, **caractérisée en ce que** les troubles sont des troubles fonctionnels ou inflammatoires des voies intestinales inférieures chez les mammifères et l'homme qui impliquent une sensibilité accrue à la douleur et/ou le passage altéré des selles dans la région du côlon.

**16.** Composés selon la revendication 14, **caractérisée en ce que** le trouble est le SCI.

**17.** Procédé de préparation des composés de formule générale I

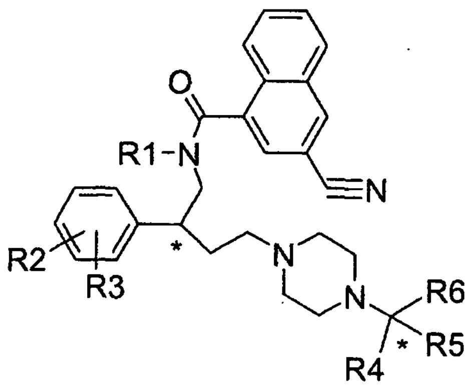

I

dans laquelle

R1 est choisi dans le groupe constitué par hydrogène et alkyle en $C_1$ à $C_4$,
R2 est halogène,
R3 est halogène,
R4 est choisi dans le groupe constitué par : 2-furanyle, 3-furanyle, 2-thiophène, 3-thiophène, phényle, benzyle, 2-benzofuranyle, 3-benzofuranyle, 5-chloro-2-thiophène, 4-méthylphényle, 3,4-méthylènedioxyphényle, 2-méthoxyphényle, 4-méthoxyphényle, 2-pyridinyle, 3-pyridinyle, 1-benzo [c] thiophène, 4-benzo[c]thiophène, 5-benzo [c] thiophène, 2-benzo [b] thiophène, 3-benzo [b] thiophène, 4-benzo [b] thiophène, 5-benzo [b] thiophène, 6-benzo [b] thiophène, 7-benzo [b] thiophène, 1-benzo [1,3] dioxole, 4-benzo [1,3] dioxole, et 5-benzo [1,3] dioxole,
R5 est choisi dans le groupe constitué par hydrogène et R6,
R6 représente un sous-groupe de formule générale

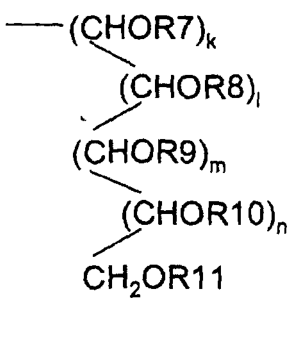

dans laquelle

R7 est choisi dans le groupe constitué par hydrogène ou alcanoyle en $C_1$ à $C_4$, ou conjointement avec un autre substituant, choisi parmi le groupe constitué par R8, R9, R10 et R11, peut former un cycle à 5 ou 6 chaînons ponté par carbonyle ou par méthylène éventuellement substitué par alkyle en $C_1$ à $C_4$ ou alkylène en $C_4$ à $C_5$,
R8 est choisi dans le groupe constitué par hydrogène ou alcanoyle en $C_1$ à $C_4$, ou conjointement avec un autre

substituant, choisi parmi le groupe constitué par R7 R9, R10 et R11, peut former un cycle à 5 ou 6 chaînons ponté par carbonyle ou par méthylène éventuellement substitué par alkyle en $C_1$ à $C_4$ ou alkylène en $C_4$ à $C_5$.
R9 est choisi dans le groupe constitué par hydrogène ou alcanoyle en $C_1$ à $C_4$, ou conjointement avec un autre substituant, choisi parmi le groupe constitué par R7 R8, R10 et R11, peut former un cycle à 5 ou 6 chaînons ponté par carbonyle ou par méthylène éventuellement substitué par alkyle en $C_1$ à $C_4$ ou alkylène en $C_4$ à $C_5$,
R10 est choisi dans le groupe constitué par hydrogène ou alcanoyle en $C_1$ à $C_4$, ou conjointement avec un autre substituant, choisi parmi le groupe constitué par R7 R8, R9 et R11, peut former un cycle à 5 ou 6 chaînons ponté par carbonyle ou par méthylène éventuellement substitué par alkyle en $C_1$ à $C_4$ ou alkylène en $C_4$ à $C_5$,
R11 est choisi dans le groupe constitué par hydrogène ou alcanoyle en $C_1$ à $C_4$, ou conjointement avec un autre substituant, choisi parmi le groupe constitué par R7 R8, R9 et R10, peut former un cycle à 5 ou 6 chaînons ponté par carbonyle ou par méthylène éventuellement substitué par alkyle en $C_1$ à $C_4$ ou alkylène en $C_4$ à $C_5$,
k est 0 ou 1,
1 est 0 ou 1,
m est 0 ou 1,
n est 0 ou 1,

ou des sels d'addition d'acide physiologiquement compatibles de ceux-ci
**caractérisé en ce que** :

(a) un composé de formule générale VII

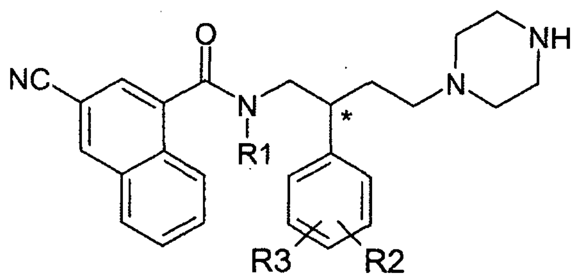

VII

est mis en réaction avec un composé de formule générale VIII,

R4 - $B(OH)_2$ VIII

et un composé de formule générale IX

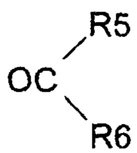

IX

pour conduire à un composé de formule générale I ; ou
(b) un composé de formule générale XV

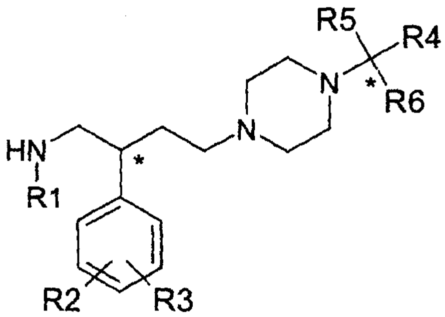

XV

est mis en réaction avec un composé de formule III

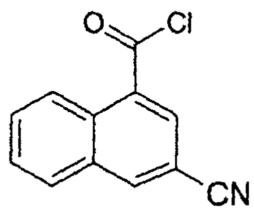

III

pour conduire à un composé de formule générale I ; ou

(c) un composé de formule générale XVII

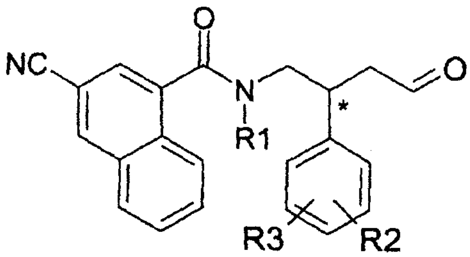

XVII

est mis en réaction avec un composé de formule générale XII

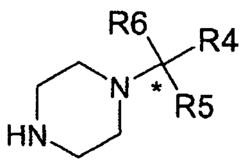

XII

pour conduire à un composé de formule générale I ;

qui est éventuellement transformé en son sel d'addition d'acide physiologiquement compatible.

**18.** Composés de formule générale VII

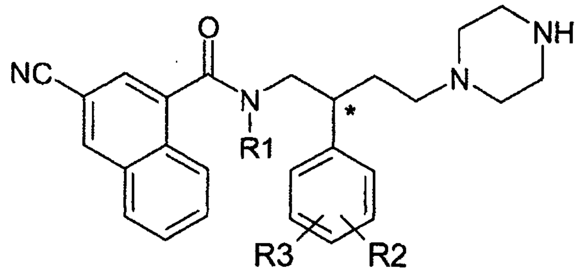

VII

dans laquelle

R1 est choisi dans le groupe constitué par hydrogène et alkyle en $C_1$ à $C_4$,
R2 est halogène, et
R3 est halogène.

**19.** Composés selon la revendication 18, **caractérisés en ce que** R1 est méthyle et **en ce que** R2 et R3 représentent chacun le chlore.

**20.** Un composé selon l'une quelconque des revendications 1 à 12 destiné à une utilisation dans une méthode d'inhibition d'un effet médié par la tachykinine chez un mammifère.

**21.** Composé selon la revendication 20, **caractérisée en ce que** la tachykinine est une neurokinine et le récepteur de la tachykinine est un récepteur de $NK_1$ ou $NK_2$.

**22.** Composé selon la revendication 20, **caractérisée en ce que** l'effet médié par la tachykinine est choisi dans le groupe constitué par l'hypotension, la bronchoconstriction et la motilité du côlon.

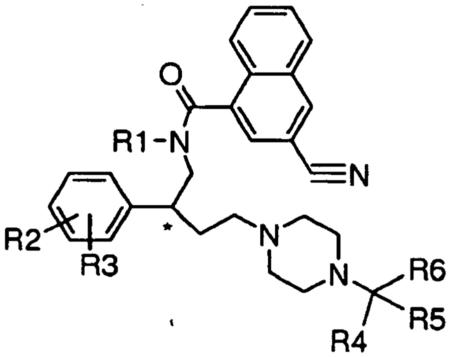

I

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004033428 A1
- EP 1293506 A1
- WO 9610568 A
- WO 9800398 A
- WO 0024510 A
- EP 0474561 A1


### Non-patent literature cited in the description


- **N.A. PETASIS et al.** *Journal of the American Chemical Society,* 1998, vol. 120, 11798-11799
- **J.A.W. MCOMIE.** Protective Groups in Organic Chemistry. Plenum Press, 1973
- **T.W. GREEN ; P.G. WUTS.** Protective Groups in Organic Synthesis. Wiley and Sons, 1999
- **W.G. THOMPSON et al.** *Gastroenterology International,* 1989, vol. 2, 92-95
- **W.G. THOMPSON et al.** *GUT,* 1999, vol. 45/II, II43-II47
- **TAKEDA, Y. ; CHOU, K. B. ; TAKEDA, J. ; SACHAIS, B.S. ; KRAUSE, J. E.** *Biochemical and Biophysical Research Communications,* 1991, vol. 179 (3), 1232-1240
- **N.P. GERARD et al.** *Journal of Biological Chemistry,* 1990, vol. 265 (33), 20455-20462